# EUROPEAN PATENT APPLICATION

(11) **EP 4 744 605 A2**
(43) Date of publication of application: **20.05.2026**
(21) Application number: 25216704.4
(22) Date of filing: 18.11.2025
(51) Int. Cl.: A61B 18/00

(54) **FILTER LIFE IN SURGICAL SMOKE EVACUATION SYSTEMS**

(30) Priority: 18.11.2024 US 202418950801
(71) Applicant: Cilag GmbH International, 6300 Zug (CH)
(72) Inventor: OBERKIRCHER, Brendan J., Cincinnati, 45242 (US); MESSERLY, Jeffrey D., Cincinnati, 45242 (US); DERRICO, Matthew E., Cincinnati, 45242 (US); ELMER, Lucas B., Cincinnati, 45242 (US); BUSH, Corey S., Cincinnati, 45242 (US); LEUCK, Stephen M., Cincinnati, 45242 (US); WRIGHT, Lindsey, Cincinnati, 45242 (US); DENZINGER, Kristen G., Cincinnati, 45242 (US); GERSEY, Stephen D., Cincinnati, 45242 (US); SCHLEY, Nicholas D., Cincinnati, 45242 (US)
(74) Representative: Carpmaels & Ransford LLP

(57) **Abstract**

A system is disclosed including an evacuator module and a controller. The evacuator module provides a housing that defines an inlet port and an outlet port, a flow path extending between the inlet and outlet ports, a pump drivable by a motor to draw smoke into the inlet port and along the flow path, and an air quality sensor positioned at a location along the flow path to measure a parameter. The controller is in operable communication with the air quality sensor and the motor. The controller is operable to control the motor based on the parameter measured by the air quality sensor.

## Description

### BACKGROUND

The present disclosure relates to surgical systems and, more particularly, smoke evacuation systems used in surgical systems.

During surgical procedure involving an energy device, smoke can be generated at the surgical site. Surgical smoke evacuators are configured to evacuate smoke, as well as fluid and/or particulate, from the surgical site.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of a computer-implemented interactive surgical system, in accordance with at least one aspect of the present disclosure.
FIG. 2 is a diagram of various modules, including an energy module and an evacuator module, and other components that are combinable to customize modular energy systems, in accordance with at least one aspect of the present disclosure.
FIG. 3 is the energy module of FIG. 2 and various surgical instruments usable therewith, in accordance with at least one aspect of the present disclosure.
FIG. 4A is a first illustrative modular energy system configuration including a header module and a display screen that renders a graphical user interface (GUI) for relaying information regarding modules connected to the header module, in accordance with at least one aspect of the present disclosure.
FIG. 4B is an isometric view of the modular energy system shown in FIG. 4A mounted to a cart, in accordance with at least one aspect of the present disclosure.
FIG. 5 is a second illustrative modular energy system configuration including a header module, a display screen, two energy modules, and an evacuator module connected together and mounted to a cart, in accordance with at least one aspect of the present disclosure.
FIG. 6 is a block diagram of a hub configuration of a modular energy system, in accordance with at least one aspect of the present disclosure.
FIG. 7 is perspective view of the evacuator module of FIG. 2, in accordance with at least one aspect of the present disclosure.
FIG. 8 is a schematic depicting internal components within the evacuator module of FIG. 7, in accordance with at least one aspect of the present disclosure.
FIG. 9 is the schematic of the evacuator module of FIG. 8 further including air quality sensors, in accordance with at least one aspect of the present disclosure.
FIG. 10 is a flow diagram for dynamically controlling a motor of the evacuator module of FIG. 9 using an air quality sensor, in accordance with at least one aspect of the present disclosure.
FIG. 11 is a table correlating flow levels of a motor to threshold volumetric flow rates of particulate, in accordance with at least one aspect of the present disclosure.
FIG. 12 is a flow diagram for measuring the quality of air through the evacuator module of FIG. 9 using an air quality sensor, in accordance with at least one aspect of the present disclosure.
FIG. 13 is a schematic of a controller receiving data from various data sources to determine a type of tissue being operated on by a surgical instrument, in accordance with at least one aspect of the present disclosure.
FIG. 14 is a schematic diagram of an evacuator module including a three-way valve, in accordance with at least one aspect of the present disclosure.
FIG. 15 is a flow diagram for determining the quality of a filter in the evacuator module of FIG. 14, in accordance with at least one aspect of the present disclosure.
FIG. 16 is a flow diagram for measuring filter life, in accordance with at least one aspect of the present disclosure
FIG. 17 illustrates a filter in accordance with at least one aspect of the present disclosure.
FIG. 18 illustrates an interior surface of an inlet layer of the filter of FIG. 17 that includes a plate in a stowed position, in accordance with at least one aspect of the present disclosure.
FIG. 19 illustrates an interior surface of an outlet layer of the filter of FIG. 17 that includes a plate in a stowed position, in accordance with at least one aspect of the present disclosure.
FIG. 20 is a schematic, cross-sectional view of the filter of FIG. 18 with the plates of the interior and exterior layers of FIGS. 18 and 19 in their respective stowed states, in accordance with at least one aspect of the present disclosure.
FIG. 21 is the schematic, cross-sectional view of FIG. 20 with the plates moved to their respective deployed states, in accordance with at least one aspect of the present disclosure.
FIG. 22 is a schematic of an evacuator module including a filter and a scale for measuring the weight of the filter, in accordance with at least one aspect of the present disclosure.
FIG. 23 is a flow diagram for dynamically controlling a speed of a motor of an evacuator module, in accordance with at least one aspect of the present disclosure.
FIG. 24 is a modular surgical system including an evacuation module and an insufflation module that are fluidically coupled to a patient, in accordance with at least one aspect of the present disclosure.

### DETAILED DESCRIPTION

Applicant of the present application owns the following U.S. Patent Applications filed concurrently herewith, the disclosure of each of which is hereby incorporated by reference in their entirety herein:
U.S. Patent Application No. 18/950,899, titled AIR MANAGEMENT IN SURGICAL SMOKE EVACUATION SYSTEMS;
U.S. Patent Application No. 18/951,268, titled INTELLIGENT INSUFFLATION AND SMOKE EVACUATION; and
U.S. Patent Application No. 18/951,342, titled SETTING EVACUATION MOTOR SPEEDS FOR SURGICAL TOOL EVACUATION MODULES.

The present disclosure relates to energy devices and surgical evacuation systems for evacuating smoke and/or other fluids and/or particulates from a surgical site.

Smoke is often generated during a surgical procedure that utilizes one or more energy devices. Energy devices use energy to affect (treat) tissue. In an energy device, the energy is supplied by a generator. Energy devices include devices with tissue-contacting electrodes, such as an electrosurgical device having one or more radio frequency (RF) electrodes, and devices with vibrating surfaces, such as an ultrasonic device having an ultrasonic blade. For an electrosurgical device, a generator is configured to generate oscillating electric currents to energize the electrodes. For an ultrasonic device, a generator is configured to generate ultrasonic vibrations to energize the ultrasonic blade. Generators are further described herein. An evacuator module is utilized to control an amount of smoke generated by the energy devices during use thereof.

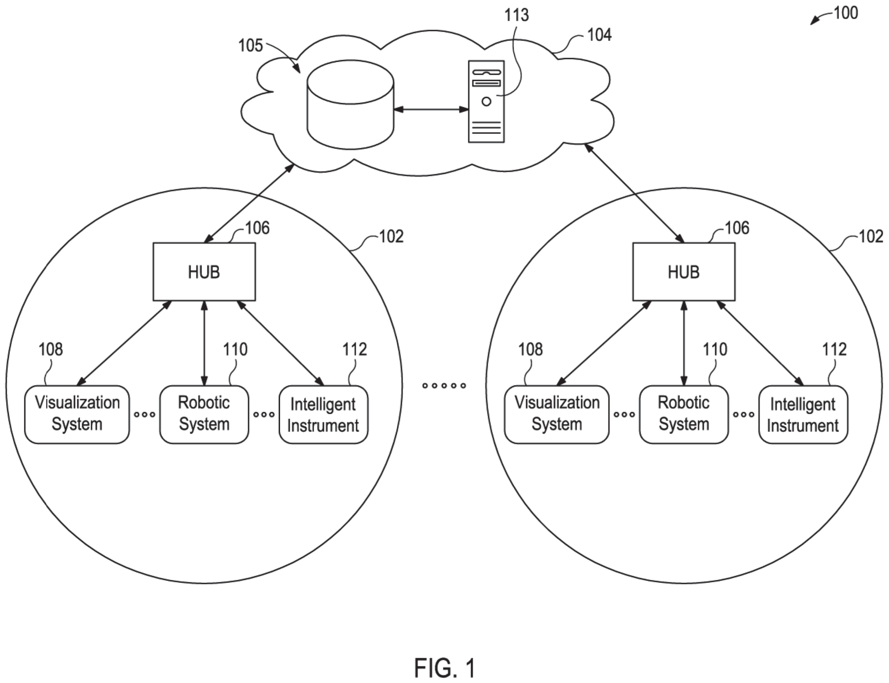

FIG. 1 is a block diagram of a computer-implemented interactive surgical system 100 (hereinafter "the surgical system 100") that may be used in accordance with at least one aspect of the present disclosure. The surgical system 100 includes one or more sub-surgical systems 102 and a cloud-based system (e.g., the cloud 104) that may include a remote server 113 in communication with a storage device 105. Each sub-surgical system 102 includes at least one surgical hub 106 in communication with the cloud 104 that may include a remote server 113.

In one example, as illustrated in FIG. 1, each sub-surgical system 102 includes a visualization system 108, a robotic system 110, and a handheld intelligent surgical instrument 112, which are configured to communicate with one another and/or the hub 106. In some aspects, each sub-surgical system 102 may include an M number of hubs 106, an N number of visualization systems 108, an O number of robotic systems 110, and a P number of handheld intelligent surgical instruments 112, where M, N, O, and P are integers greater than or equal to one. The surgical system 100 is described in more detail in U.S. Patent No. 11,666,368, entitled "METHOD FOR CONSTRUCTING AND USING A MODULAR SURGICAL ENERGY SYSTEM WITH MULTIPLE DEVICES", issued June 6, 2023, and is hereby incorporated by reference in its entirety herein.

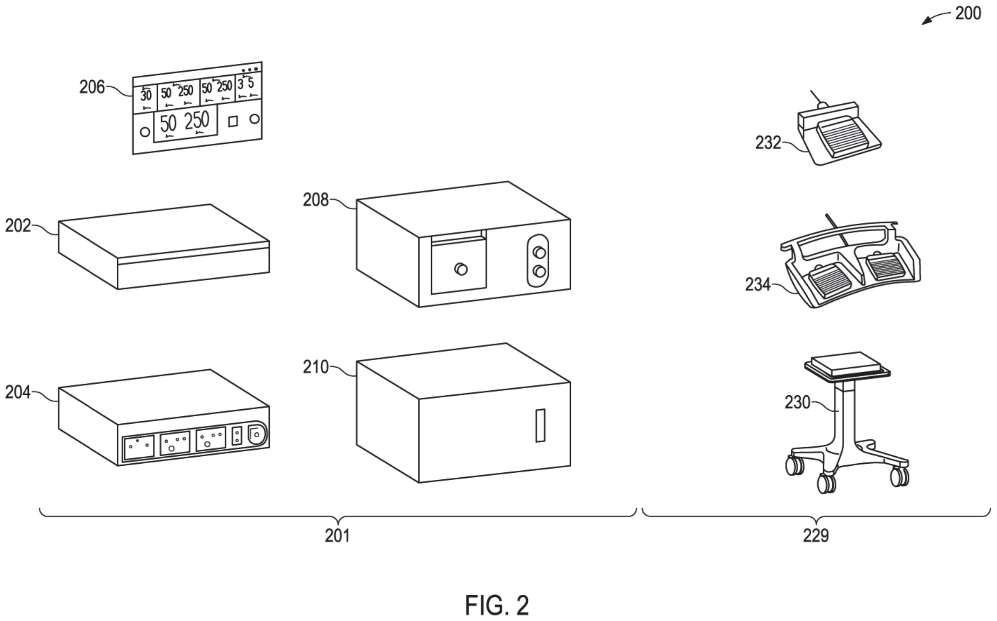

Referring now to FIG. 2, an example surgical hub 106 (FIG. 1) can be embodied as a modular energy system 200 that can include a variety of different modules 201 that are connectable together in a stacked configuration. In one aspect, the modules 201 can be both physically and communicably coupled together when stacked or otherwise connected together into a singular assembly. Further, the modules 201 can be interchangeably connectable together in different combinations or arrangements. In one aspect, each of the modules 201 can include a consistent or universal array of connectors disposed along their upper and lower surfaces, thereby allowing any module 201 to be connected to another module 201 in any arrangement (except that, in some aspects, a particular module type, such as the header module 202, can be configured to serve as the uppermost module within the stack, for example). In an alternative aspect, the modular energy system 200 can include a housing that is configured to receive and retain the modules 201. The modular energy system 200 can also include a variety of different components or accessories that are also connectable to or otherwise associatable with the modules 201.

The modular energy system 200 can be assembled from a variety of different modules 201, some examples of which are illustrated in FIG. 2. Each of the different types of modules 201 can provide different functionality, thereby allowing the modular energy system 200 to be assembled into different configurations to customize the functions and capabilities of the modular energy system 200 (e.g., by customizing the modules 201 that are included in each modular energy system 200). The modules 201 of the modular energy system 200 can include, for example, a header module 202 (which can include a display screen 206), an energy module 204, an evacuator module 208, and a visualization module 210.

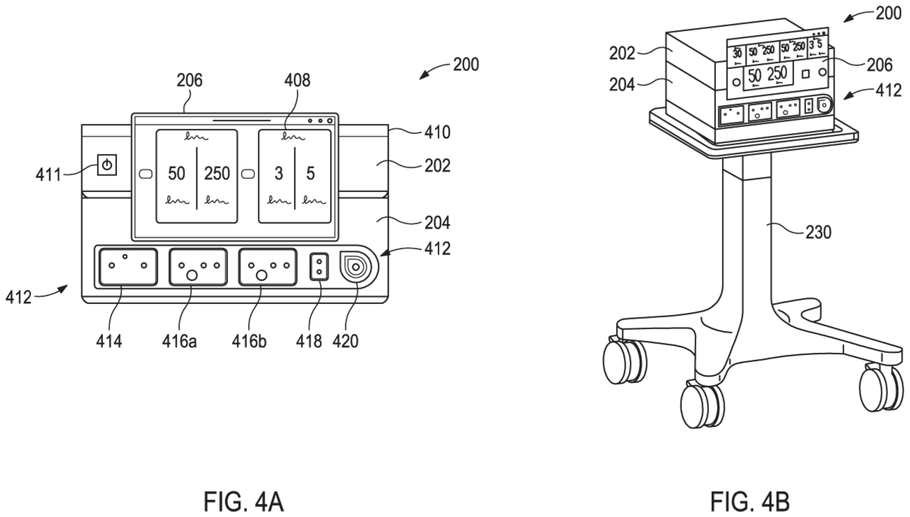

In the depicted aspect, the header module 202 is configured to serve as the top or uppermost module within the modular energy system stack and can thus lack connectors along its top surface. In another aspect, the header module 202 can be configured to be positioned at the bottom or the lowermost module within the modular energy system stack (*i.e*., a "footer" module) and can thus lack connectors along its bottom surface. In yet another aspect, the header module 202 can be configured to be positioned at an intermediate position within the modular energy system stack and can thus include connectors along both its bottom and top surfaces. The header module 202 can be configured to control the system-wide settings of each module 201 and component connected thereto through physical controls 411 (FIG. 4A) thereon and/or a graphical user interface (GUI) 408 (FIG. 4A) rendered on the display screen 206. Such settings could include the activation of the modular energy system 200, the volume of alerts, the footswitch settings, the settings icons, the appearance or configuration of the user interface, the surgeon profile logged into the modular energy system 200, and/or the type of surgical procedure being performed. The header module 202 can also be configured to provide communications, processing, and/or power for the modules 201 that are connected to the header module 202.

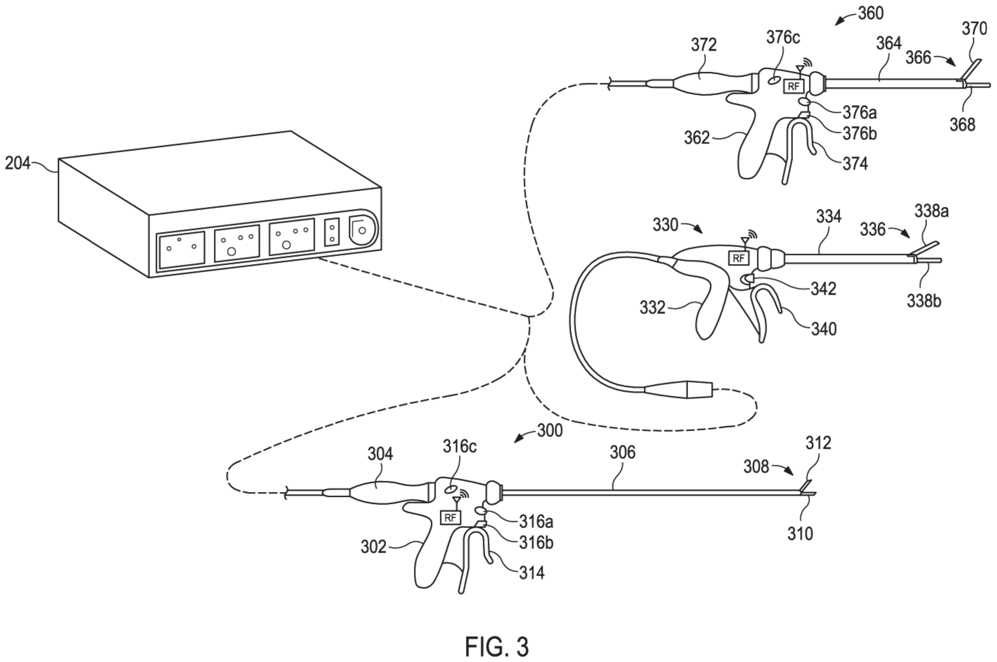

The energy module 204, alternately referred to as a generator module, can be configured to generate one or multiple energy modalities for driving electrosurgical and/or ultrasonic surgical instruments connected thereto. For example, referring to FIG. 3, the generator 204 is configured to drive multiple surgical instruments 300, 330, 360. The first surgical instrument is an ultrasonic surgical instrument 300 and comprises a handpiece 302 (HP), an ultrasonic transducer 304, a shaft 306, and an end effector 308. The end effector 308 comprises an ultrasonic blade 310 acoustically coupled to the ultrasonic transducer 304 and a clamp arm 312. The handpiece 302 comprises a trigger 314 to operate the clamp arm 312 and a combination of toggle buttons 316a, 316b, 316c to energize and drive the ultrasonic blade 310 or other function. The toggle buttons 316a-c can be configured to energize the ultrasonic transducer 304 with the generator 204.

The generator 204 is also configured to drive the second surgical instrument 330, which is an RF electrosurgical instrument and comprises a handpiece 332 (HP), a shaft 334, and an end effector 336. The end effector 336 comprises electrodes in clamp arms 338a, 338b and return through an electrical conductor portion of the shaft 334. The electrodes are coupled to and energized by a bipolar energy source within the generator 204. The handpiece 332 includes a trigger 340 manually actuatable to operate the clamp arms 338a,b, and an energy button 342 to actuate an energy switch to energize the electrodes in the end effector 336.

The generator 204 is also configured to drive the third surgical instrument 360, which is a multifunction surgical instrument 360 and comprises a handpiece 362 (HP), a shaft 364, and an end effector 366. The end effector 366 comprises an ultrasonic blade 368 and a clamp arm 370. The ultrasonic blade 368 is acoustically coupled to an ultrasonic transducer 372. The handpiece 362 includes a trigger 374 to operate the clamp arm 370, and a combination of toggle buttons 376a, 376b, 376c to energize and drive the ultrasonic blade 368 or other function. The toggle buttons 376a-c can be configured to energize the ultrasonic transducer 372 with the generator 204 and energize the ultrasonic blade 368 with a bipolar energy source also contained within the generator 204. Further aspects of the surgical instruments are described in U.S. Patent No. 10,624,691, entitled "TECHNIQUES FOR OPERATING GENERATOR FOR DIGITALLY GENERATING ELECTRICAL SIGNAL WAVEFORMS AND SURGICAL INSTRUMENTS", issued April 21, 2020, which is herein incorporated by reference in its entirety herein.

The evacuator module 208 (FIG. 2) can be configured to evacuate smoke, fluid, and/or particulates generated by the application of therapeutic energy to the tissue by one or more of the surgical instruments 300, 330, 360. Example evacuator modules are described in more detail elsewhere herein, as well as in U.S. Patent No. 11,602,393, entitled "SURGICAL EVACUATION SENSING AND GENERATOR CONTROL", issued March 14, 2023, which is hereby incorporated by reference in its entirety herein.

The visualization module 210 (FIG. 2) can be configured to interface with visualization devices (i.e., scopes) and accordingly provide increased visualization capabilities. Example visualization modules and systems are described in more detail in U.S. Patent No. 11,284,963, entitled "METHOD OF USING IMAGING DEVICES IN SURGERY", issued March 29, 2022, which is hereby incorporated by reference in its entirety herein.

Referring again to FIG. 2, the modular energy system 200 can further include a variety of accessories 229 that are connectable to the modules 201 for controlling the functions thereof or that are otherwise configured to work in conjunction with the modular energy system 200. The accessories 229 can include, for example, a single-pedal footswitch 232, a dual-pedal footswitch 234, and a cart 230 for supporting the modular energy system 200 thereon. The footswitches 232, 234 can be configured to control the activation or function of particular energy modalities output by the energy module 204, for example.

By utilizing modular components, the depicted modular energy system 200 provides a surgical platform that grows with the availability of technology and is customizable to the needs of the facility and/or surgeons. Further, the modular energy system 200 supports combo devices (e.g., dual electrosurgical and ultrasonic energy generators) and supports software-driven algorithms for customized tissue effects. Still further, the surgical system architecture reduces the capital footprint by combining multiple technologies critical for surgery into a single system.

The various modular components utilizable in connection with the modular energy system 200 can include monopolar energy generators, bipolar energy generators, dual electrosurgical/ultrasonic energy generators, display screens, and various other modules and/or other components described elsewhere herein.

Referring now to FIG. 4A, the header module 202 can, in some aspects, include the display screen 206 that renders a GUI 408 for relaying information regarding the modules 201 (FIG. 2) connected to the header module 202. In some aspects, the GUI 408 of the display screen 206 can provide a consolidated point of control of all of the modules 201 making up the particular configuration of the modular energy system 200. In alternative aspects, the header module 202 can lack the display screen 206, or the display screen 206 can be detachably connected (removably attachable) to a housing 410 of the header module 202. In such aspects, the header module 202 can be communicably couplable to an external system that is configured to display the information generated by the modules 201 of the modular energy system 200. For example, in robotic surgical applications, the modular energy system 200 can be communicably couplable to a robotic cart or robotic control console, which is configured to display the information generated by the modular energy system 200 to the operator of the robotic surgical system. As another example, the modular energy system 200 can be communicably couplable to a mobile display that can be carried or secured to a surgical staff member for viewing thereby. In aspects utilizing a user interface that is separate from or otherwise distinct from the modular energy system 200, the user interface can be wirelessly connectable with the modular energy system 200 as a whole or one or more modules 201 thereof such that the user interface can display information from the connected modules 2001 thereon.

Referring still to FIG. 4A, the energy module 204 can include a port assembly 412 including (providing) a number of different ports configured to deliver different energy modalities to corresponding surgical instruments (e.g., surgical instruments 300, 330, 360 of FIG. 3, for example) that are connectable thereto. In the particular aspect illustrated in FIGS. 4A, the port assembly 412 includes a bipolar port 414, a first monopolar port 416a, a second monopolar port 416b, a neutral electrode port 418 (to which a monopolar return pad is connectable), and a combination energy port 420. However, this particular combination of ports is simply provided for illustrative purposes and alternative combinations of ports and/or energy modalities may be possible for the port assembly 412.

As noted above, the modular energy system 200 can be assembled into different configurations. Further, the different configurations of the modular energy system 200 can also be utilizable for different surgical procedure types and/or different tasks. For example, FIGS. 4A and 4B illustrate a first illustrative configuration of the modular energy system 200 including the header module 202 (including the display screen 206) and the energy module 204 connected together. Such a configuration can be suitable for laparoscopic and open surgical procedures, for example. As shown in FIG. 4B, the modular energy system 200 can be positioned on a cart 230 enabling the modular energy system 200 to be easily moved (wheeled) around the operating room, for example.

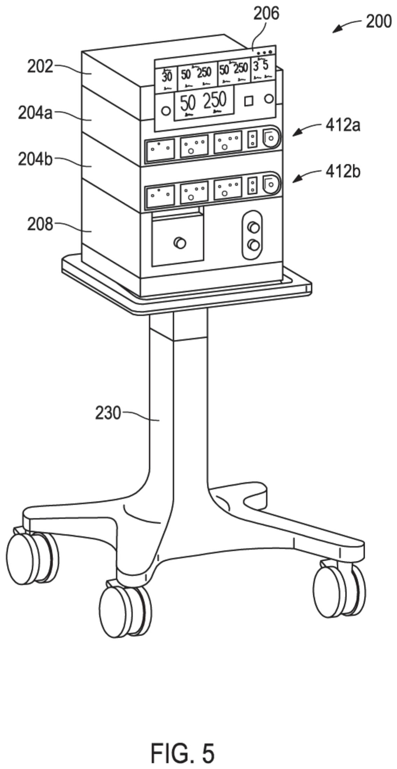

FIG. 5 illustrates a second illustrative configuration of the modular energy system 200 including the header module 202 (including the display screen 206), a first energy module 204a, a second energy module 204b, and the evacuator module 208 connected together and positioned on the cart 230. In such a configuration, the evacuator module 208 can evacuate smoke, fluid, and/or particulates generated by surgical instruments powered by the energy modules 204a,b.

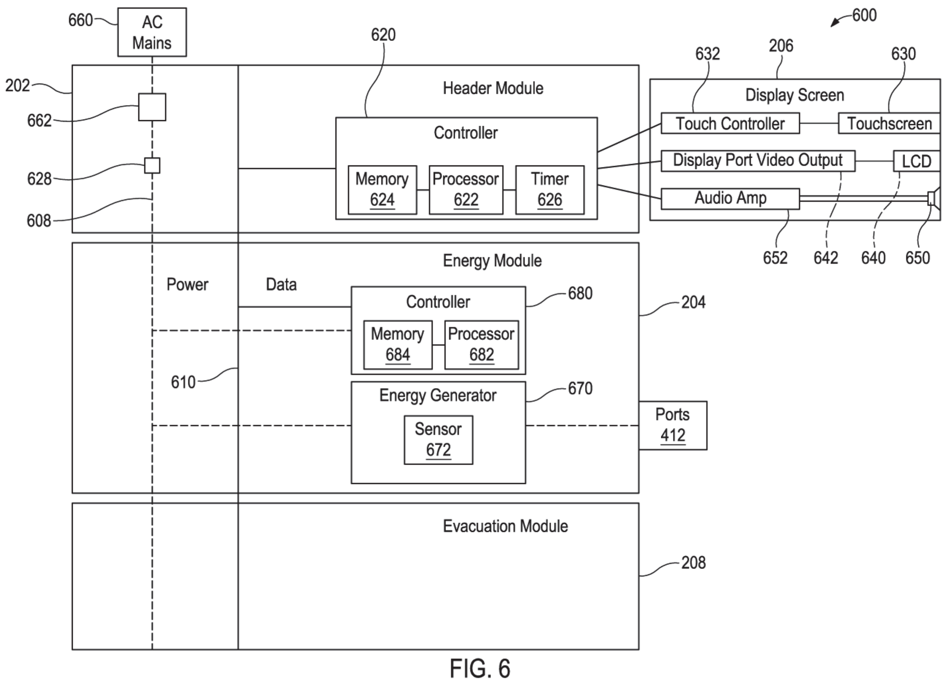

FIG. 6 is a block diagram of an example modular energy system 600, in accordance with at least one aspect of the present disclosure. As illustrated, the modular energy system 600 includes the header module 202 (including the display screen 206), the energy module 204 stacked under and coupled to the header module 202, and the evacuator module 208 stacked under and coupled to the energy module 204.

The header module 202 is configured to monitor, control, energize, and provide feedback concerning operation of the modules within the modular energy system 600, such as the energy module 204 and the evacuator module 208. As illustrated, the header module 202 includes a controller 620 that comprises a processor 622 and a memory 624 storing computer readable instructions executable by the processor 622 to carry out functions and operations of the header module 602. Examples of the memory 624 include, but are not limited to, random access memory (RAM), read-only memory (ROM), computer chips, optical discs (e.g., compact discs (CDs), digital video discs (DVDs), etc.), magnetic disks (e.g., hard disk drives (HDDs), floppy disks, ZIP^{®} disks, etc.), magnetic tape, and solid state storage devices (e.g., memory cards, "flash" media, etc.). As used herein, the term "computer readable medium" refers to any device or system for storing and providing information (e.g., data and instructions) to the processor 622. Examples of computer readable media include, but are not limited to, optical discs, magnetic disks, magnetic tape, solid-state media, and servers for streaming media over networks.

Based on instructions stored in the memory 624, the processor 622 may be configured to control power and data transmissions between the header module 202, the energy module 204, and the evacuator module 208 through a power interface 608 and a data interface 610. For example, the header module 202 can transmit various commands to the energy module 204 and evacuator module 208 (through the energy module 204) via the data interface 610. Such commands can be based on user inputs received at the display screen 206 or inputs received by the controller 620 from various sensors communicably coupled to the modular energy system 600, as discussed elsewhere herein.

As a further example, power may be transmitted to the energy module 204 and the evacuation module 208 (through the energy module 204) from the header module 202 via the power interface 608. The header module 202 may receive power from an external power source 660 (referred to herein as "AC Mains"), such as a wall outlet, for example. The header module 202 may include an AC/DC converter 662 which receives the AC power from the AC Mains 660 and converts the AC power to DC power. The controller 202 may then distribute the DC power to the energy module 204 and the evacuation module 208. The controller 620 may further include a timer 626 for measuring elapsed time. The header module 202 may include a sensor 628, such as a current sensor and/or a power sensor, for example, in operable communication with the controller 620 for measuring current and power along the power interface 608.

As shown in FIG. 6, the energy module 204 may include a controller 680 that comprises a processor 682 and a memory 684 storing computer readable instructions executable by the processor 682 to carry out functions and operations of the energy module 204. The processor 682 and a memory 684 may be similar to processor 622 and memory 624, respectively. The controller 680 may receive power from the AC/DC converter 662 along the power interface 608 and may be in operable communication with controller 620 via the data interface 610.

The energy module 204 may further include an energy generator 670. The energy generator 670 may receive power from the AC/DC converter 662 along the power interface 608 and may be in operable communication with controller 680, such as via a wired or wireless connection. The energy generator 670 may be operable to provide therapeutic energy to one or more surgical instruments, such as the surgical instruments 300, 330, 360, via the port assembly 412, such as via the bipolar port 414 (FIG. 4), the first or second monopolar ports 416a, 416b (FIG. 4), or the combination energy port 420 (FIG. 4), for example. For instance, the energy generator 670 may be energized with DC power provided thereto from the AC/DC converter 662 along the power interface 608. The controller 680 may then receive an input, such as from the controller 620. Based on the input, the controller 680 may control the energy generator 670 to provide therapeutic energy to one or more surgical instruments coupled to the energy module 204 at the port assembly 412. The energy generator 670 may include a sensor 672, such as a current sensor and/or a power sensor, for example, in operable communication with the controller 680 for measuring current and/or power provided by the energy generator 670. The sensor 672 may also comprise an impedance sensor for measuring the impedance of tissue grasped by one of the surgical instruments.

As shown in FIG. 6, the display screen 206 includes a touchscreen 630 coupled to a touch controller 632. The touch controller 630 is coupled to the controller 620 to read inputs, such as user inputs, from the touchscreen 630. The controller 620 drives an LCD display 640 through a display/port video output signal 642. The controller 620 is further coupled to an audio amplifier 652 to drive one or more speakers 650.

### Surgical Smoke Evacuation

As provided herein, energy devices, such as the energy delivery devices 300, 330, 360 of FIG. 3, deliver mechanical (ultrasonic, for example) and/or electrical (RF, for example) energy to target tissue in order to treat the tissue (e.g. to cut the tissue, cauterize blood vessels and/or coagulate the tissue within and/or near the targeted tissue). The cutting, cauterization, and/or coagulation of tissue can result in fluids and/or particulates being released into the air. Such fluids and/or particulates emitted during a surgical procedure can constitute smoke, for example, which can comprise carbon particles and/or other particles suspended in air. In other words, a fluid can comprise smoke and/or other fluidic matter.

Approximately 90% of endoscopic and open surgical procedures generate some level of smoke. The smoke can be unpleasant to the olfactory senses of the clinician(s), the assistant(s), and/or the patient(s), may obstruct the clinician(s)'s view of the surgical site, and may be unhealthy to inhale in certain instances. For example, smoke generated during an electrosurgical procedure can contain toxic chemicals including acrolein, acetonitrile, acrylonitrile, acetylene, alkyl benzenes, benzene, butadiene, butene, carbon monoxide, creosols, ethane, ethylene, formaldehyde, free radicals, hydrogen cyanide, isobutene, methane, phenol, polycyclic aromatic hydrocarbons, propene, propylene, pyridene, pyrrole, styrene, toluene, and xylene, as well as dead and live cellular material (including blood fragments), and viruses. Certain materials identified in surgical smoke have been classified as containing known carcinogens. It is estimated that one gram of tissue cauterized during an electrosurgical procedure can be equivalent to the toxins and carcinogens of six unfiltered cigarettes. Additionally, exposure to the smoke released during an electrosurgical procedure has been reported to cause eye and lung irritation to health care workers.

In addition to the toxicity and odors associated with the material in surgical smoke, the size of particulate in surgical smoke can be harmful to the respiratory system of the clinician(s), the assistant(s), and/or the patient(s). In certain instances, the particulates can be extremely small. Repeated inhalation of extremely small particulate can lead to acute and chronic respiratory conditions in certain instances.

Many electrosurgical systems employ a surgical evacuation system that draws in and captures the resultant smoke from a surgical procedure, and directs the captured smoke through a filter and an exhaust port away from the clinician(s) and/or from the patient(s). For example, an evacuation system, such as the evacuator module 208 (FIG. 2), can be configured to evacuate smoke that is generated during an electrosurgical procedure. Such an evacuation system can be referred to as a "smoke evacuation system," though such evacuation systems can be configured to evacuate more than just smoke from a surgical site.

Throughout the present disclosure, the "smoke" evacuated by an evacuation system is not limited to just smoke. Rather, the smoke evacuation systems disclosed herein can be used to evacuate a variety of fluids, including liquids, gases, vapors, smoke, steam, or combinations thereon. The fluids can be biologic in origin and/or can be introduced to the surgical site from an external source during a procedure. The fluids can include water, saline, lymph, blood, exudate, and/or pyogenic discharge, for example. Moreover, the fluids can include particulates or other matter (e.g. cellular matter or debris) that is evacuated by the evacuation system. For example, such particulates can be suspended in the fluid.

### VOC Sensing Data Point for Surgical Smoke Evacuation

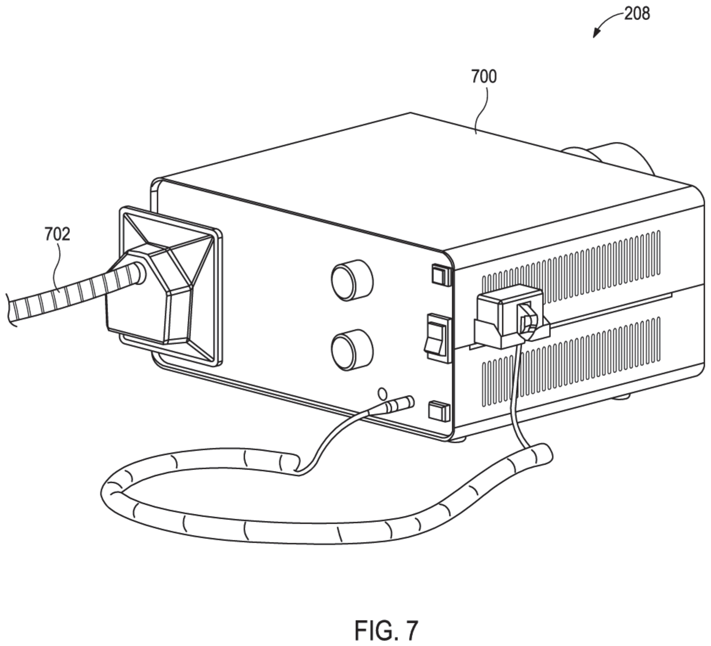

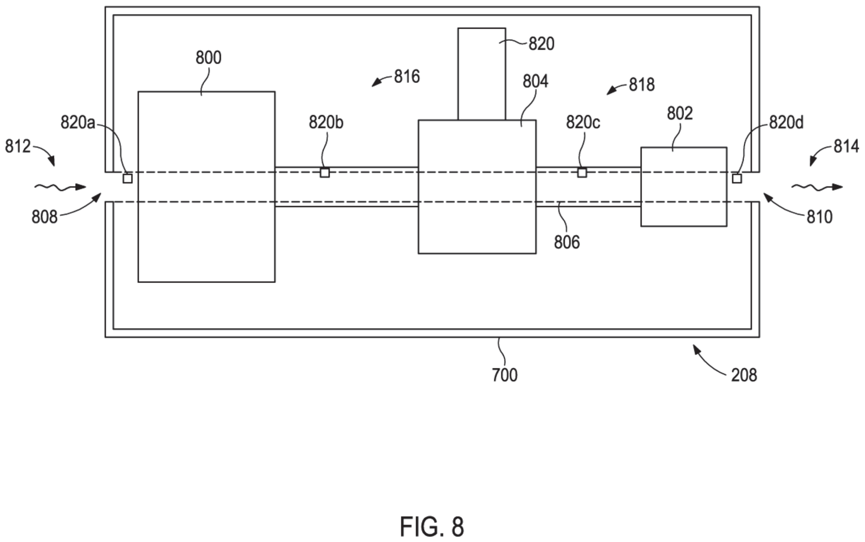

FIG. 7 is a detailed view of the evacuator module 208 of FIG. 2, according to at least one aspect of the present disclosure. The evacuator module 208 includes an evacuator housing 700 and a suction conduit 702. As described in more detail below, in one or more embodiments, a fan or pump 804 (FIG. 8) and a filter 800 (FIG. 8) may be positioned within the evacuator housing 700. Smoke drawn into the evacuator housing 700 travels to the filter 800 via the suction conduit 702, and harmful toxins and offensive smells are filtered out of the smoke as it moves through (traverses) the filter 800. The suction conduit 702 can also be referred to as a vacuum and/or evacuation conduit and/or tube. Filtered air 814 (FIG. 8) may then exit the evacuator module 208 as exhaust.

Referring now to FIG. 8, various internal components of the evacuator module 208 housed within the evacuator housing 700 are schematically depicted. As noted above, the evacuator module 208 may include a filter 800, an exhaust mechanism 802, and a fan or pump 804. The evacuator module 208 defines a flow path 806 (shown in dashed lines) extending through the evacuator housing 700 and having an inlet port 808 and an outlet port 810. The filter 800, the pump 804, and the exhaust mechanism 802 are sequentially arranged in-line within the flow path 806 through the evacuator housing 700 between the inlet port 808 and the outlet port 810. The inlet port 808 can be fluidically coupled to the suction conduit 702 (FIG. 7), which can comprise a distal conduit opening positionable at a surgical site.

The pump 804 is configured to produce a pressure differential in the flow path 806 by a mechanical action. The pressure differential is configured to draw smoke 812 from the surgical site into the inlet port 808 and along the flow path 806. After moving through the filter 800, the smoke 812 can be considered "filtered" smoke or air 814 (referred to herein as "filtered air 814"), which can continue through the flow path 806 and is eventually expelled (discharged) through the outlet port 810.

As illustrated, the flow path 806 may include a first zone 816 and a second zone 818. The first zone 816 is located upstream from the pump 804; the second zone 818 is located downstream from the pump 804. The pump 804 is configured to generate the vacuum and otherwise pressurize the fluid in the flow path 806 to drive the fluid from the first zone 816 to the second zone 818 through the pump 804. A motor 820 drives the pump 804. The exhaust mechanism 802 is a mechanism that can control the velocity, the direction, and/or other properties of the filtered air 814 exiting the evacuator module 208 at the outlet port 810.

The flow path 806 through the evacuator module 208 can be comprised of a tube or other conduit that substantially contains and/or isolates the fluid moving through the flow path 806 from the fluid (the ambient environment) outside the flow path 806. For example, the first zone 816 of the flow path 806 can comprise a tube through which the flow path 806 extends between the filter 800 and the pump 804. The second zone 818 of the flow path 806 can also comprise a tube (conduit) through which the flow path 806 extends between the pump 804 and the exhaust mechanism 802. The flow path 806 also extends through the filter 800, the pump 804, and the exhaust mechanism 802 so that the flow path 806 extends continuously from the inlet port 808 to the outlet port 810.

In operation, the smoke 812 can flow into the filter 800 after traversing the inlet port 808 and can be pumped through the flow path 806 by the pump 804 such that the smoke 812 is drawn into the filter 800. The filtered air 814 discharged from the filter 800 can then be pumped through the exhaust mechanism 802 and out the outlet port 810 of the evacuator module 208. The filtered air 814 exiting the evacuator module 208 at the outlet port 810 is the exhaust, and can consist of filtered gases that have passed through the evacuator module 208. Additional information regarding the evacuator module 208 is described in U.S. Patent No. 11,602,393, entitled "SURGICAL EVACUATION SENSING AND GENERATOR CONTROL", issued March 14, 2023, which is hereby incorporated by reference in its entirety herein.

According to embodiments of the present disclosure, the evacuator module 208 may further include a plurality of sensors 820a, 820b, 820c, 820d positioned along the flow path 806 for measuring one or more parameters associated with the smoke 812 and/or the filtered air 814 flowing along the flow path 806. When the evacuator module 208 is in a stacked configuration with a header module 202, such as in the configuration shown in FIG. 6, for example, the sensors 820a-d can be in operable communication with the controller 620, such as along the data interface 610 (FIG. 6), and can receive power from the header module 202 via the power interface 608 (FIG. 6). The controller 620 may receive measurements from the sensors 820a-d and control various operations of the modular surgical system based on these measurements. For example, in some embodiments, the controller 620 can control a speed of the motor 820 based on the received measurements.

One or more of the sensors 820a-d may comprise flow sensors for measuring a flow rate of the smoke 812/filtered air 814 along the flow path 806, such as a flow rate entering the inlet port 808 (sensor 820a), a flow rate through the first zone 816 (sensor 820b), a flow rate through the second zone 818 (sensor 820c), and/or a flow rate exiting the outlet port 810 (sensor 820d), for example. Alternatively, or in addition thereto, one or more of the sensors 820a-d may comprise pressure sensors for measuring a pressure of smoke 812/filtered air 814 along the flow path 806, such as at the inlet port 808 (sensor 820a), the first zone 816 (sensor 820b), the second zone 818 (sensor 820c), and/or at the exit outlet port 810 (sensor 820d), for example. In some embodiments, the sensors 820a-d may be used by the controller 620 to measure a pressure differential along the flow path 806, such as a pressure differential across the filter 800 using the first and second sensors 820a,b. The evacuator module 208 may include a combination of both flow sensors and pressure sensors.

Over time, filters in smoke evacuators, such as the filter 800 in the evacuator module 208, become saturated with airborne particles and their effectiveness may correspondingly diminish, necessitating timely replacement to maintain the performance of the smoke evacuator. Current methods for determining when to replace the filter 800 may not adequately quantify the amount of particulate that has passed through the filter, which can result in the filter being replaced too early or too late. Accordingly, improved systems and methods for determining when to replace a filter in an evacuator module are desired.

In use, a clinician may activate a surgical instrument, such as one of surgical instruments 300, 330, 360 (FIG. 3), to cut a target tissue. Based on the surgical instrument being activated, the controller 620 (FIG. 6) can control the motor 820 (and therefore, the pump 804) to drive smoke 812 generated by the surgical instrument through the evacuator module 208, while also initiating the timer 626 (FIG. 6) to measure an elapsed time (in seconds, for example) that the surgical instrument is activated. In addition, the controller 620 can receive real-time data (measurements) from the sensor(s) 820a-d in the evacuator module 208 to determine the flow rate of smoke 812 (measured in cm³/second or m³/second, for example) that passes through the filter 800. Utilizing the determined time of activation and the detected rate of smoke 812, the controller 620 may calculate (determine) the volume of smoke 812 (measured in cm³ or m³, for example) that has been drawn through the filter 800. The determined volume of smoke 812, however, may not accurately represent the amount of particulate that has been captured by the filter 800.

For example, during one procedure, a surgical instrument may be activated for a first amount of time and the controller 620 may determine that a first volume of smoke 812 passed through the filter 800 during that time. During a second surgical procedure, a surgical instrument may be activated for a second amount of time (identical to the first amount of time) and the controller 620 may determine that a second volume of smoke (identical to the second volume of smoke) passed through the filter 800 during that time. However, during the first procedure, a first amount of particulate was generated by the surgical instrument, whereas during the second procedure, a second amount of particulate different (greater or less) than the first amount of particulate was generated. While the controller 620 determined that the volume of smoke 812 through the filter 800 between the first and second procedures was identical, the amount of particulate actually captured by the filter 800 was different. Measuring the quantity of airborne particulate that passes to the filter 800 may provide the controller 620, as well as the user, with a better understanding as to when the filter 800 should be replaced in the evacuator module 208.

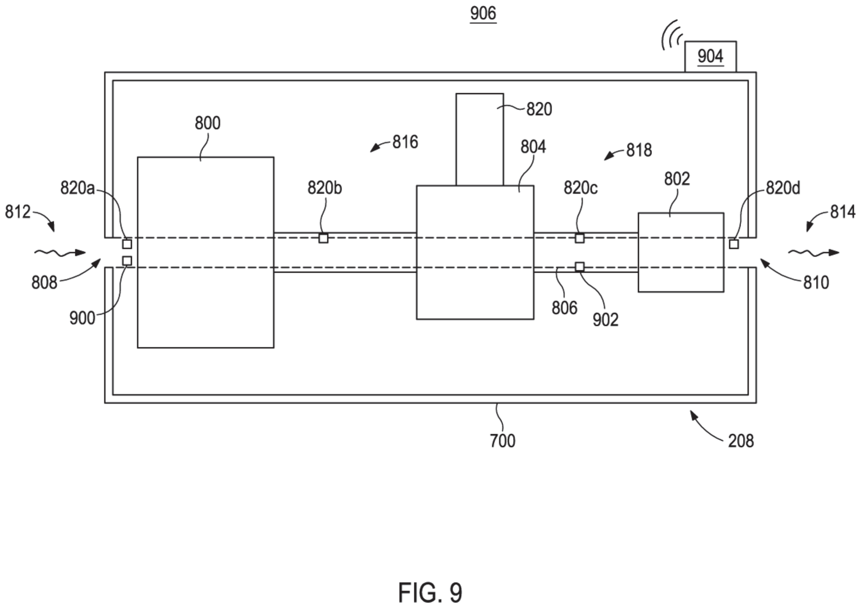

Referring now to FIG. 9, the evacuator module 208 may further include an air quality sensor 900 for measuring a parameter, such as an amount of particulate in the smoke 812. The air quality sensor 900 may be arranged upstream of the filter 800 and along the flow path 806. In some applications, the air quality sensor 900 may be arranged at or near the inlet port 808, but could alternatively be coupled to the filter 800. In at least one embodiment, the air quality sensor 900 may be arranged along a tube in the flow path 806 arranged intermediate the inlet port 800 and the filter 800.

The air quality sensor 900 may be a volatile organic compound (VOC) sensor, an air quality index (AQI) sensor, a carbon monoxide (CO) sensor, a PM2.5 sensor, or a PM10 sensor, or combinations thereof. When the evacuator module 208 is in a stacked configuration with a header module 202, such as in the configuration shown in FIG. 6, for example, the air quality sensor 900 can be in operable communication with the controller 620 (FIG. 6) of the header module 202 (FIG. 6), such as along the data interface 610 (FIG. 6), and can receive power from the header module 202 via the power interface 608 (FIG. 6).

In some embodiments, the controller 620 (FIG. 6) may calculate an amount of particulate that has passed to the filter 800 based on measurements obtained from the sensors. For example, during a procedure, a surgical instrument may be activated for an amount of time and the controller 620 may determine (using the timer 626 (FIG. 6) and the sensors 820a-d, for example) the volume of smoke 812 that passed through the filter 800 during that time, as discussed elsewhere herein. Using measurements from the air quality sensor 900, the controller 620 (FIG. 6) may further calculate the amount of particulate passing to the filter 800 during that time. In some aspects, the calculated amount of particulate corresponds to the amount of particulate present in the determined volume of smoke 812. This additional data enables the controller 620 (FIG. 6) to better understand the condition of the filter 800 over time and, thus, increase the ability of the controller 620 (FIG. 6) to know when the filter 800 should be replaced.

For instance, during one procedure, the determined volume of smoke 812 may include a relatively low (first amount) of particulate, whereas during a second procedure, the determined volume of smoke 812 may include a relatively large amount (second amount greater than the first amount) of particulate. Utilizing the air quality sensor 900 enables the controller 620 to better understand (determine) the amount of particulate accumulated by the filter 800 for a given procedure, which enables the controller 620 to more accurately determine when the filter 800 should be replaced.

The filter 800 may lose a desired effectiveness after a threshold value (amount) of particulate has been accumulated thereon. In some embodiments, the threshold value can be stored in the memory 624 (FIG. 6). The controller 620 (FIG. 6) may monitor the amount of particulate that passes to the filter 800 over time, as described above, and compare the monitored (real-time) particulate amount to the threshold amount value. Based on the monitored amount of particulate that passed to the filter 800 approaching, reaching, or exceeding the threshold value, the controller 620 (FIG. 6) can generate an alert to inform a user that the filter 800 should be replaced. For example, in some embodiments, the alert comprises a visual alert via the LCD 640 (FIG. 6) or an audio alert via the speaker 650 (FIG. 6). The controller 620 may receive measurements from the air quality sensor 900, as well as from the sensors 820a-d, and control various operations of the modular surgical system based on the received measurements.

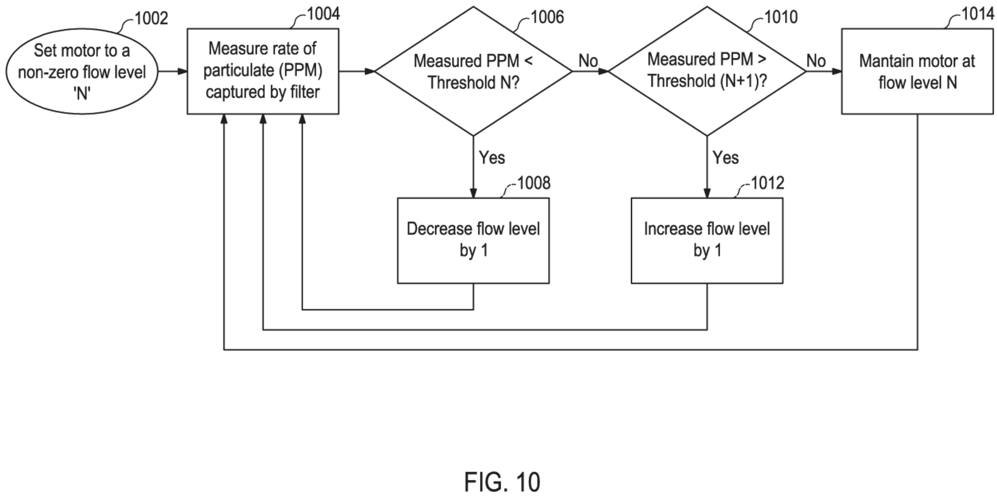

Referring now to FIG. 10, a flow diagram 1000 for dynamically controlling a speed of the motor 820 (FIGS. 8-9) is provided, according to at least one aspect of the present disclosure. In some embodiments, the flow diagram 1000 is embodied as an algorithm stored in the memory 624 (FIG. 6) of the controller 620 (FIG. 6), and is executable by the processor 622 (FIG. 6). In some embodiments, the algorithm is executable by the processor 622 (FIG. 6) based on the motor 820 (FIG. 9) being actuated by the controller 620 (FIG. 6), such as in response to a surgical instrument being activated to cut tissue.

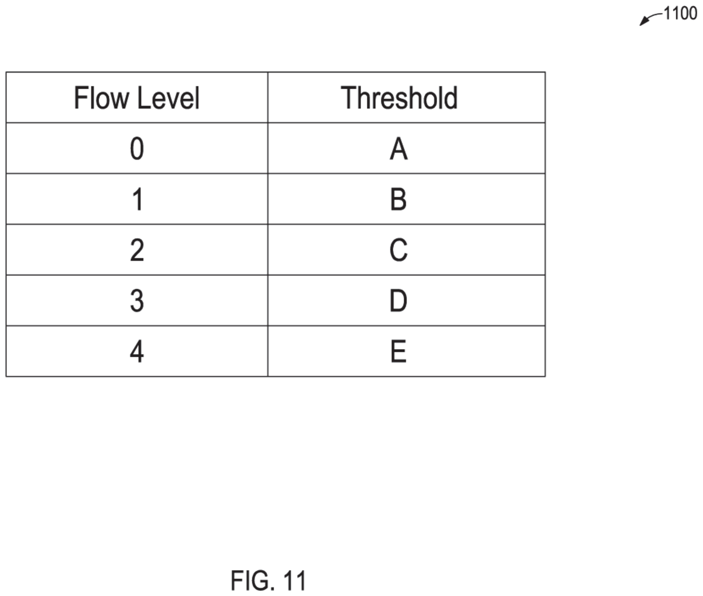

FIG. 11 provides a table 1100 that correlates flow levels of the motor 820 (FIG. 9) to threshold volumetric flow rates of particulate. In some embodiments, the table 1100 is stored in the memory 624 (FIG. 6). In some embodiments, the flow level of the motor 820 (FIG. 9) corresponds to a speed of the motor 820 (FIG. 9). In some embodiments, a flow level of 0 corresponds to the motor 820 being in an "off" state where the motor 820 does not drive (abstains from driving) the pump 804 to drive smoke 812 through the evacuator module 208. In some embodiments, a flow level of 1, 2, 3, or 4 corresponds to the motor 820 being in an "on" state where the motor 820 drives the pump 804 to drive smoke 812 through the evacuator module 208 at progressively faster speeds. For example, a higher flow level corresponds to a faster motor 820 speed (*i.e.,* flow level (speed) 4 > level 3 > level 2 > level 1). In some embodiments, threshold rate E > rate D > rate C > rate B > rate A. While five flow levels and five corresponding threshold rates are shown and described, various other embodiments are envisioned including fewer flow levels/threshold rates (such as two, three, or four, for example) or more flow levels/threshold rates (such as six, seven, or eight, for example).

Example operation of the evacuator module 208 is now provided with reference to the algorithm 1000 of FIG. 10, and with continued reference to FIGS. 6, 9, and 11. In example operation, a user may activate a surgical instrument, such as one of surgical instruments 300, 330, 360 (FIG. 3), to cut a target tissue. Based on the surgical instrument being activated, the controller 620 can execute the algorithm 1000, beginning at step 1002. At step 1002, the controller 620 controls the motor 820 (and therefore, the pump 804) to drive (draw) smoke 812 generated by the surgical instrument through (into) the evacuator module 208. More specifically, the controller 620 transitions the motor 820 from the "off" state (flow level 0) to an "on" state at flow level 'N' (where N is any one of flow levels 1-4). The controller 620 may transition the motor 820 from flow level 0 to the lowest flow level (lowest speed, level 1), an intermediate flow level (intermediate speed, levels 2 or 3), or the highest flow level (highest speed, level 4).

At step 1004, the controller 620 can receive measurements from sensors, such as the sensors 820a-d and/or the air quality sensor 900, to measure the volumetric flow rate of particulate passing to the filter 800. For example, the controller 620 may utilize elapsed time measured by the timer 626, a flow rate detected by one or more of the sensors 820a-d, and the amount of particulate measured by the air quality sensor 900 to determine the volumetric flow rate of particulate passing to the filter 800. The controller 620 may continuously measure the volumetric flow rate, or may intermittently measure the volumetric flow rate, such as at predefined intervals or at predefined times. The controller 620 may measure the volumetric flow rate based on a user input, such as at the touchscreen 630.

At step 1006, the controller 620 compares the volumetric flow measured rate to a corresponding threshold volumetric flow rate, as defined in table 1100 for the flow level N set at step 1002. For example, at step 1002, the controller 620 may set the motor 820 to run at flow level 2, which has a corresponding threshold rate level of C (as defined in table 1100). Consequently, at step 1006, the controller 620 compares the measured volumetric flow rate to the threshold volumetric flow rate of C.

Based on the controller 620 determining that the measured volumetric flow rate is less than the corresponding threshold volumetric flow rate, the controller 620 may proceed along the 'Yes' path to step 1008 and decrease the set flow level N by 1 (*i.e.,* N-1). For example, at step 1002, the controller 620 may have set the motor to run at flow level 2, which has a corresponding threshold volumetric flow rate level of C. At step 1006, the controller 620 may determine the volumetric flow rate is a rate less than the threshold rate of C. Accordingly, the controller 620 proceeds to step 1008 and transitions the motor 620 from flow level 2 to flow level 1. The controller 620 then proceeds back to step 1004 to measure the volumetric flow rate of particulate passing to the filter 800.

Based on the controller 620 determining that the measured volumetric flow rate of particulate is greater than the corresponding threshold volumetric flow rate, the controller 620 may proceed along the 'No' path to step 1010 and compare the measured volumetric flow rate to the corresponding threshold volumetric flow rate for flow level N+1, as defined in table 1100. For example, at step 1002, the controller 620 may have set the motor to run at flow level 2, which has a corresponding threshold volumetric flow rate level of C. At step 1006, the controller 620 may determine the volumetric flow rate is a rate greater than the threshold volumetric flow rate of C. Accordingly, the controller 620 proceeds to step 1010 and compares the measured volumetric flow rate to the corresponding threshold level for flow level 3, as defined in table 1100.

Based on the controller 620 determining that the measured volumetric flow rate is greater than the corresponding threshold volumetric flow rate for flow rate N+1, the controller 620 may proceed along the 'Yes' path to step 1012 and increases the set flow level by 1 (*i.e.* N+1). For example, at step 1002, the controller 620 may have set the motor to run at flow level 2, which has a corresponding threshold volumetric flow rate level of C. At step 1010, the controller 620 may determine the measured volumetric flow rate is a rate greater than threshold volumetric flow rate of D (threshold volumetric flow rate for flow level 3). Accordingly, the controller 620 proceeds to step 1012 and transitions the motor 820 from flow level 2 to flow level 3. The controller 620 then proceeds back to step 1004 to measure the volumetric flow rate of particulate passing to the filter 800.

Based on the controller 620 determining that the measured volumetric flow rate is less than the corresponding threshold volumetric flow rate for flow rate N+1, the controller 620 may proceed along the 'No' path to step 1014 and maintain the motor 820 at flow level N. For example, at step 1002, the controller 620 may have set the motor to run at flow level 2 at step 1002, which has a corresponding threshold volumetric flow rate level of C. At step 1010, the controller 620 may determine the measured volumetric flow rate is a rate less than threshold volumetric flow rate of D (threshold volumetric flow rate for flow level 3). Accordingly, the controller 620 proceeds to step 1014 and maintains the motor 820 at flow level 2. The controller 620 then proceeds back to step 1004 to measure the volumetric flow rate of particulate passing to the filter 800.

The foregoing algorithm 1000 allows the controller 620 to dynamically control the speed of the motor 820 over time, and according to a real-time measured volumetric flow rate of particulate passing to the filter 800. In some embodiments, the controller 620 may measure an amount of particulate passing to the filter 800 over time (as tracked by the sensors 820a-d, 900, for example). In such embodiments, the table 1100 may correlate flow levels (speeds) of the motor 820 to threshold amounts of particulate that passed to the filter 800. Accordingly, utilizing the algorithm 1000, the controller 620 can dynamically adjust the motor 820 over time according to a measured amount of particulate that passed to the filter 800. For example, the controller 620 can dynamically increase the speed of the motor 820 as the amount of particulate that passed to the filter 800 increases over time.

Referring again to FIG. 9, the evacuator module 208 can further include a second air quality sensor 902 for measuring a parameter, such as an amount of particulate in the filtered air 814. The air quality sensor 900 can be coupled to the pump 804, the exhaust mechanism 802, the outlet port 810, or to any portion of the flow path 806 downstream from the filter 800, such as to a tube thereof in the first zone 816 or the second zone 818, for example. The air quality sensor 902 may be similar to the air quality sensor 900.

When the evacuator module 208 is in a stacked configuration with a header module 202, such as in the configuration shown in FIG. 6, for example, the air quality sensor 902 can be in operable communication with the controller 620 (FIG. 6) of the header module 202 (FIG. 6), such as along the data interface 610 (FIG. 6), and can receive power from the header module 202 via the power interface 608 (FIG. 6). The controller 620 (FIG. 6) may receive measurements from the air quality sensor 902 to determine an amount of particulate in the filtered air 814. For example, during a procedure, a surgical instrument may be activated for an amount of time and the controller 620 may determine, using the timer 626 (FIG. 6) and the flow sensors 820b-d, for example, the volume of filtered air 814 through the evacuator module 208. Using measurements from the air quality sensor 902, the controller 620 may further calculate the amount of particulate present in the filtered air 814 during that time. In some aspects, the calculated amount of particulate corresponds to the amount of particulate present in the determined volume of filtered air 814. This additional data enables the controller 620 to better understand the condition of the filter 800 over time and, thus, increase the ability of the controller 620 (FIG. 6) to know when the filter 800 should be replaced.

For example, the filter 800 may lose a desired effectiveness over time, resulting in particulate potentially passing through the filter 800 and into the filtered air 814. The controller 620 (FIG. 6) can monitor the amount of particulate in the filtered air 814 over time, as described above, and compare the measured volumetric flow rate and/or amount of entrained particulate to a threshold value. Based on the monitored amount and/or volumetric flow rate of particulate present in the filtered air 814 approaching, reaching, or exceeding the threshold value, the controller 620 can generate an alert to inform the user that the filter 800 should be replaced. For example, in some embodiments, the alert comprises a visual alert via the LCD 640 (FIG. 6) or an audio alert via the speaker 650 (FIG. 6). The threshold value may be stored in the memory 624 (FIG. 6). The controller 620 may receive measurements from the second air quality sensor 902, as well as from the first air quality sensor 900 and/or the sensors 820a-d, and control various operations of the modular surgical system based on the received measurements.

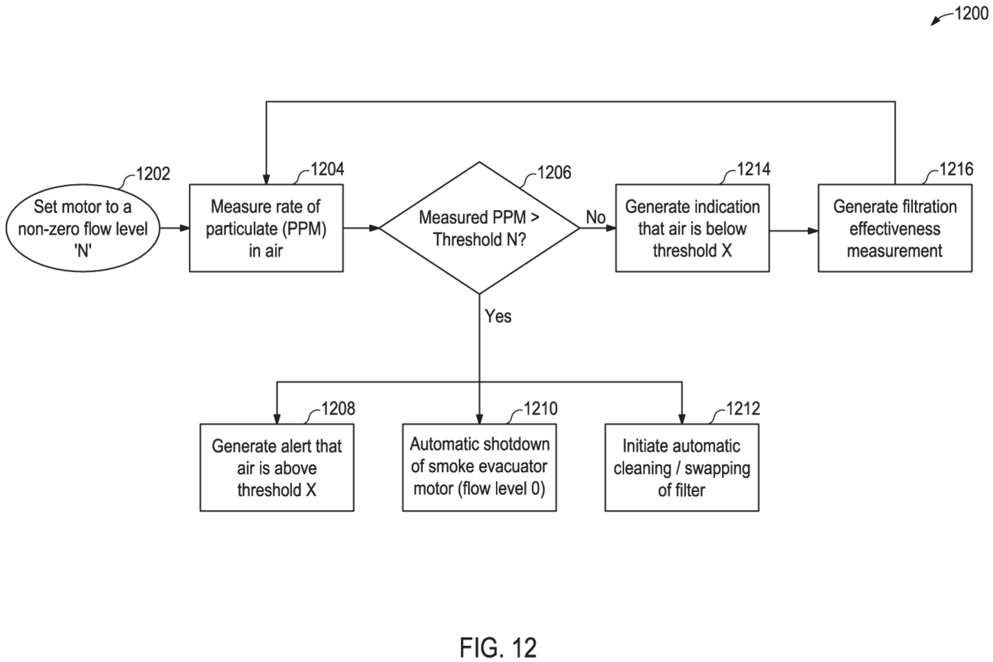

Referring now to FIG. 12, a flow diagram 1200 is provided, according to at least one aspect of the present disclosure. In some embodiments, the flow diagram 1200 is embodied as an algorithm, stored in the memory 624 (FIG. 6) of the controller 620 (FIG. 6), and is executable by the processor 622 (FIG. 6). In some embodiments, the algorithm is executable by the processor 622 based on the motor 820 (FIG. 8) being actuated by the controller 620 (FIG. 6), such as in response to a surgical instrument being activated to cut tissue.

Example operation of the evacuator module 208 is now provided with reference to FIG. 12, and continued reference to FIGS. 6 and 9. In operation, a user can activate a surgical instrument, such as one of surgical instruments 300, 330, 360 (FIG. 3), to cut a target tissue. Based on the surgical instrument being activated, the controller 620 can execute the algorithm 1200, beginning at step 1202. At step 1202, the controller 620 controls the motor 820 (and therefore, the pump 804) to drive smoke 812 generated by the surgical instrument through the evacuator module 208. More specifically, the controller 620 transitions from the motor 820 from an "off" state (flow level 0) to an "on" state at flow level 'N' (where N is any one of flow levels 1-4). The controller 620 may transition the motor 820 from flow level 0 to the lowest flow level (lowest speed, level 1), an intermediate flow level (intermediate speed, levels 2 or 3), or the highest flow level (highest speed, level 4).

At step 1204, as the controller 620 controls the motor 820 to drive smoke 812 through the evacuator module 208, the controller 620 can receive measurements from sensors, such as the sensors 820b-d and/or the air quality sensor 902, to measure the volumetric flow rate of particulate in the filtered air 814 through the evacuator module 208. For example, the controller 620 can utilize elapsed time measured by the timer 626, a flow rate detected by one or more of the sensors 820b-d, and the amount of particulate measured by the air quality sensor 902 to determine the volumetric flow rate of particulate in the filtered air 814 through the evacuator module 208. The controller 620 may continuously measure the volumetric flow rate, or may intermittently measure the volumetric flow rate, such as at a predefined rate or at predefined times (intervals). The controller 620 may measure the volumetric flow rate based on a user input, such as at the touchscreen 630.

At step 1206, the controller 620 compares the measured volumetric flow rate to a threshold volumetric flow rate, which may be stored in the memory 624. Based on the controller 620 determining that the measured volumetric flow rate is greater than the threshold volumetric flow rate, the controller 620 may proceed along the 'Yes' path to steps 1208, 1210, and 1212. At step 1208, the controller 620 can generate an alert, such as a visual alert on the LCD 640 and/or an audio alert with the speaker 650, to inform a user that the measured volumetric flow rate of particulate in the filtered air 814 is greater than the threshold volumetric flow rate. Furthermore, at step 1210, the controller 620 can automatically shut down the motor 820 to prevent the motor 820 from driving the pump 804. For instance, the controller 620 may transition the motor 820 to flow level 0. In other embodiments, the controller 620 may further shut down the energy module 204 (FIG. 2) to prevent the surgical instrument from generating any additional smoke 812 until the filter 800 has been replaced. Furthermore, at step 1212, the controller 620 can initiate an automatic cleaning procedure for the filter 800 and/or automatically release the filter 800 from the evacuator module 208 for the clinician to remove and replace.

Based on the controller 620 determining that the measured volumetric flow rate is less than the threshold volumetric flow rate, the controller 620 may proceed along the 'No' path to step 1214. At step 1214, the controller 620 can generate an indication, such as a visual indication on the LCD 640 and/or an audio indication with the speaker 650, to inform a user that the measured volumetric flow rate of particulate in the filtered air 814 is less than the threshold volumetric flow rate. The controller 620 may then proceed to step 1216 to generate an effectiveness measurement of the filter 800, which may be defined as the ability of the filter 800 to operate at a useful and efficient level. For example, the controller 620 may calculate the effectiveness of the filter 800 with the following equation: Efficiency % = 100 - 100*(volumetric flow rate of particulate in the filtered air 814 / volumetric flow rate of particulate in the smoke 812). The controller 620 may then proceed back to step 1204 to continue measuring the volumetric flow rate of particulate in the filtered air 814.

The foregoing algorithm 1200 allows the controller 620 to dynamically measure the volumetric flow rate particulate in the filtered air 814 and perform various functions, such as shutting down the motor 820 based on a measured volumetric flow rate of particulate approaching, reaching, or exceeding a threshold volumetric flow rate, for example. In some embodiments, the controller 620 may measure an amount of particulate present in the filtered air 814, compare the measured amount of particulate to a particulate threshold, and perform the various functions based on the measured amount of particulate approaching, reaching, or exceeding the threshold. In such embodiments, utilizing the algorithm 1200, the controller 620 can perform various functions according to a measured amount of particulate in the filtered air 814, regardless of the volumetric flow rate of filtered air 814 that passes through evacuator module 208.

Referring again to FIG. 9, the evacuator module 208 may further include a third air quality sensor 904 for measuring the amount of particulate in an operating room 906 where the evacuator module 208 is positioned. The air quality sensor 904 may be in operable communication with the controller 620 (FIG. 6), such as wired or wirelessly. The controller 620 may receive measurements from the third air quality sensor 904 and may shut down the motor 820 and/or the energy module 206 based on the measured amount of particulate in the operating room 906 approaching, reaching, or exceeding a threshold value, which may be stored in the memory 624 (FIG. 6). The controller 620 may generate an indication, such as a visual indication on the LCD 640 and/or an audio indication with the speaker 650, to inform a user of the measured amount of particulate in the operating room 906. In some embodiments, inclusion of the third air quality sensor 904 may enable the controller 620 to better understand the type of tissue that is being operated on by a surgical instrument.

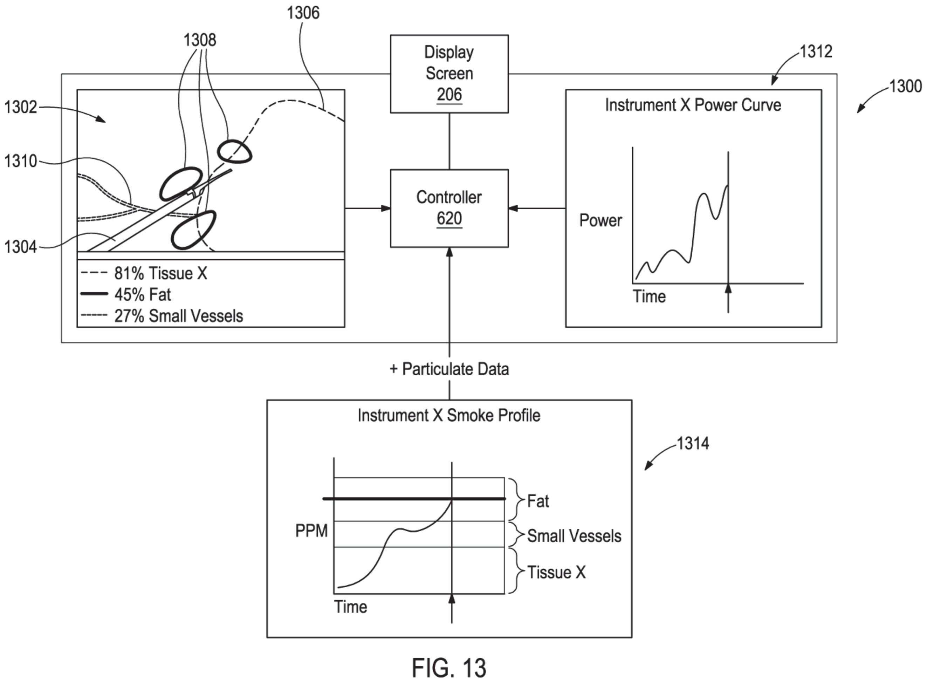

FIG. 13 is a schematic diagram illustrating the controller 620 of the header module 202 (FIG. 2) receiving data from various data sources to determine a type of tissue being operated on by a surgical instrument, according to at least one aspect of the present disclosure. The controller 620 may be in operable communication with a visualization system, such as visualization system 108 (FIG. 1) and/or visualization module 210 (FIG. 2), to receive real-time visualization data therefrom. For example, the real-time visualization data may include an image of a surgical site 1302 that includes a surgical instrument 1304. The visualization system may be configured to identify tissue 1306, fat 1308, and/or vessels 1310, for example, at the surgical site, overlay these identified features on the image of the surgical site 1302, and provide the identified percentages of the tissue 1306, fat 1308, and/or vessels 1310 to the controller 620. Additional discussion regarding these visualization systems is provided in International Patent Pub. No. WO/2019/133125, entitled "ADJUSTMENTS BASED ON AIRBORNE PARTICLE PROPERTIES", PCT filed November 14, 2018, which is hereby incorporated by reference in its entirety herein.

While the controller 620 may have a certain level of confidence in the type of tissue being operated on based on data from the visualization system, improvements can be made to increase the confidence of the controller 620. An improved confidence in the type of tissue being operated on can allow the controller 620 to more accurately control the amount of energy provided to the surgical instrument 1304 to improve seal quality and minimize smoke generation.

The controller 620 may also be in operable communication with an energy module, such as energy module 204 (FIG. 2) that provides power to the surgical instrument 1304. The controller 620 may monitor, over time, an amount of power supplied to the surgical instrument 1304, as shown in power curve 1312. The controller 620 may monitor the power using the sensor 628 (FIG. 6).

The controller 620 may also be in operable communication with an evacuator module, such as the evacuator module 208 (FIG. 9), and receive smoke data therefrom. For instance, the evacuator module 208 can include an air quality sensor 900 (FIG. 9) that is operable to sense particulate (ppm) in smoke 812 (FIG. 9). The controller 620 may track the particulate over time, as shown in graph 1314, and determine the contents (type) of the tissue being operated on by the surgical instrument 1304, such as the amount of fat content in the tissue, based on the tracked particulate over time.

The controller 620 may compare the detected fat from the visualization data to the detected fat from the smoke data 1314 and, based on the comparison, determine the type of tissue that is being operated on by the surgical instrument 1304. For example, the controller 620 may take the average of the quantity of fat as detected by from the visualization data and the smoke data and determine the type of tissue based on the average value. In some embodiments, the memory 624 (FIG. 6) includes a look-up table that correlates detected fat quantities to types of tissue. In some embodiments, the controller 620 can receive the visualization data, the energy data, and the smoke data and determine the type of tissue being operated on by the surgical instrument 1304 according to the received data.

Accordingly, the smoke data, in combination with the visualization data and the power data, for example, can provide the controller 620 with an additional data point that can be used to identify the type of tissue being operated on by the surgical instrument 1304, improving the confidence of the controller 620. An improved confidence in the type of tissue being operated on can allow the controller 620 to more accurately control the amount of energy provided to the surgical instrument 1304 to improve seal quality and minimize smoke generation.

### Monitoring Filter Status of Smoke Evacuator through a Bypass Airflow Test

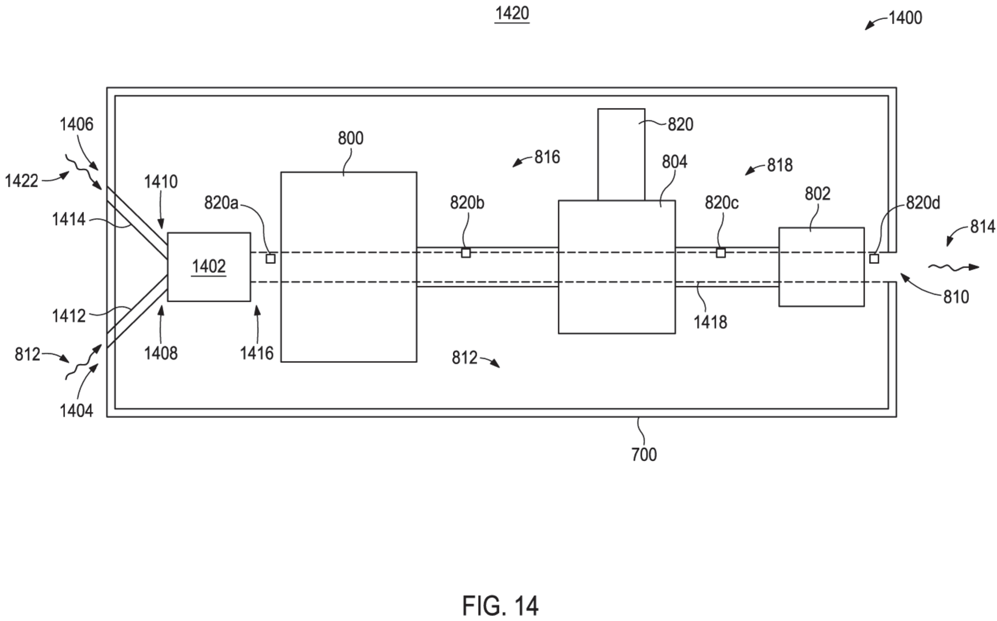

FIG. 14 is a schematic diagram of an example evacuator module 1400 including a three-way valve 1402, according to at least one aspect of the present disclosure. The evacuator module 1400 may be similar in some respects to the evacuator module 208 of FIG. 2, and therefore may be best understood with reference thereto, where like numbers used in the figures will represent similar components not described again in detail.

As illustrated, the evacuator module 1400 includes first and second inlet ports 1404, 1406 in fluid communication with first and second inlets 1408, 1410 provided on the three-way valve 1402, respectively, via first and second flow paths 1412, 1414, respectively. The first inlet port 1404 may be fluidically coupled to the suction conduit 702 (FIG. 7), which includes a distal conduit opening positionable at a surgical site. The second inlet port 1406 may be fluidically coupled to a second suction conduit, similar to the first suction conduit 702, and including a distal conduit opening positionable (arrangeable) at an ambient environment 1420 surrounding the evacuator module 1400, such as an operating room. Alternatively, as shown in FIG. 14, the second inlet port 1406 may omit a suction conduit and the inlet port 1406 may be in direct fluidic communication with the ambient environment 1420. The evacuator module 1400 further includes the outlet port 810 in fluid communication with an outlet 1416 of the three-way valve 1402 via a common flow path 1418 (shown in dashed lines).

The three-way valve 1402 can be transitioned between a first or "surgical" state and a second or "test" state. In the first state, the three-way valve 1402 places the first inlet port 1404 in fluid communication with the outlet port 810 via the first flow path 1412, the first inlet 1408 of the three-way valve 1402, the outlet 1416 of the three-way valve 1402, and the common flow path 1418. Moreover, in the first state, the three-way valve 1402 may prevent fluid communication (fluidically decouple) between the second inlet port 1406 and the outlet port 810 (*i.e*., blocking flow). In the second state, the three-way valve 1402 transitions to place the second inlet port 1406 in fluid communication with the outlet port 810 via the second flow path 1414, the second inlet 1410 of the three-way valve 1402, the outlet 1416 of the three-way valve 1402, and the common flow path 1418, while fluidically decoupling the first inlet port 1404 from the outlet port 810 (*i.e.,* blocking flow).

When the evacuator module 1400 is in a stacked configuration with a header module 202, similar to the configuration shown in FIG. 6, for example, the three-way valve 1402 can be in operable communication with the controller 620 (FIG. 6) of the header module 202, such as along the data interface 610 (FIG. 6), and can receive power from the header module 202 via the power interface 608 (FIG. 6). The controller 620 may be operable and otherwise programmed to transition the three-way valve 1402 between the first and second states, such as based on a user input provided at the touchscreen 630 (FIG. 6) or based on parameters sensed by sensors, such as from the sensors 820a-d (FIG. 8) or air quality seconds 900, 902, 904 (FIG. 9), for example. In some embodiments, the three-way valve 1402 includes a motor in operable communication with the controller 620 (FIG. 6) and any suitable valve (e.g., gate, globe, ball, etc.) that is transitionable (actuatable) between an open state and a closed state by the motor to transition the evacuator module 1400 between the surgical and test states. In some embodiments, the three-way valve 1402 comprises a solenoid valve.

The first flow path 1412, the second flow path 1414, and the common flow path 1418 through the evacuator module 208 can be comprised of tubes or other conduits that substantially contain and/or isolate the fluid moving through the flow paths 1412, 1414, 1418 from the fluid outside the flow paths 1412, 1414, 1418.

The pump 804 of the evacuator module 1400 is configured to produce a pressure differential. With the three-way valve 1402 in the first state, the pressure differential is configured to draw smoke 812 from the surgical site into the first inlet port 1404 and along the first flow path 1412, through the three-way valve 1402, and along the common flow path 1418 to the filter 800. After the smoke 812 has moved through (traverses) the filter 800, filtered air 814 is discharged and continues through the common flow path 1418 until expelled through the outlet port 810. The pump 804 is configured to create a vacuum and otherwise pressurize the fluid in the first flow path 1412 and the common flow path 1418 to drive the fluid from the first inlet port 1404, along the first zone 816 to the second zone 818 through the pump 804. The motor 820 drives the pump 804.

With the three-way valve 1402 in the second state, the pressure differential is configured to draw ambient air 1422 from the ambient environment 1420 into the second inlet port 1406 and along the second flow path 1410, through the three-way valve 1402, and along the common flow path 1418 to the filter 800. The ambient air 1422 can then move through (traverse) the filter 800, through the common flow path 1418, and be expelled through the outlet port 810.

In operation, a user can activate a surgical instrument, such as one of surgical instruments 300, 330, 360 (FIG. 3), to cut (treat) a target tissue. Based on the surgical instrument being activated, the controller 620 (FIG. 6) can place (transition) the three-way valve 1402 into the first (surgical) state and control the motor 820 (and therefore, the pump 804) to drive smoke 812 generated by the surgical instrument into the inlet port 1404 and along the first flow path 1412, through the three-way valve 1402, and along the common flow path 1418 to the filter 800. In some embodiments, the controller 620 can determine a status of the filter 800 as the pump 804 drives (draws) smoke 812 through the evacuator module 1400 using sensors 820a-d and/or air quality sensors 900, 902 (FIG. 9), as described elsewhere herein.

Alternatively, or in combination therewith, the controller 620 may determine a real-time status of the filter 800 utilizing the three-way valve 1402. For instance, at a time where the evacuator module 1400 is not being used to draw smoke 812 from a surgical site, such as prior to, during, or after a procedure, the controller 620 (FIG. 6) can transition the three-way valve 1402 to the second (test) state. The three-way valve 1402 may transition to the second state to determine the status of the filter 800 based on a user input provided at the touchscreen 630 (FIG. 6), or automatically, such when the evacuator module 1400 is powered on or a threshold amount of time has elapsed since a surgical instrument was activated, for example. The elapsed time may be measured by the timer 626 (FIG. 6) and the threshold amount of time may be stored in the memory 624 (FIG. 6).

Based on the three-way valve 1402 being transitioned to the second state, the controller 620 may control the motor 820 (and therefore, the pump 804) to drive ambient air 1422 from the ambient environment 1420 into the inlet port 1406 and along the second flow path 1414, through the three-way valve 1402, and along the common flow path 1418 to the filter 800.

As the motor 820 drives (draws) ambient air 1422 through the filter 800, the controller 620 (FIG. 6) may monitor various parameters of the evacuator module 1400 using the one or more sensors 820a-d. For example, as the motor 820 drives ambient air 1422 through the filter 800, the controller 620 (FIG. 6) may receive pressure measurements from the sensors 820a,b and determine a pressure differential across the filter 800, which may be indicative of the quality (state) of the filter 800. In some embodiments, the controller 620 may compare the determined pressure differential to a threshold pressure differential. The threshold pressure differential may be stored in the memory 624 (FIG. 6). Based on the determined pressure differential reaching or exceeding the threshold pressure differential, the controller 620 can generate an alert, such as a visual alert on the LCD 640 (FIG. 6) and/or an audio alert with the speaker 650 (FIG. 6), to inform a user that the filter 800 should be replaced. Based on the determined pressure differential reaching or exceeding the threshold pressure differential, the controller 620 may prevent activation of one or more modules in the modular surgical system, such as the energy module 204 or the evacuator module 1400, for example, until the filter 800 is replaced.

The memory 624 (FIG. 6) may include a look-up table that correlates determined pressure differentials to filter integrity. For instance, with a new filter 800 positioned in the evacuator module 1400 and ambient air 1422 being driven through the filter 800, the controller 620 (FIG. 6) can determine a first pressure differential using the first and second sensors 820a,b. The controller 620 (FIG. 6) can look-up the first pressure differential in the look-up table, which may be correlated to 100% filter integrity (*i.e.,* new filter). The controller 620 (FIG. 6) may then display this determined filter integrity on the LCD 640 (FIG. 6) such that a user can know the remaining filter integrity (filter life) of the filter 800.

At a later time, after the filter 800 has filtered an amount of smoke 812, ambient air 1422 may again be driven through the filter 800 and the controller 620 (FIG. 6) may determine a second pressure differential greater than the first pressure differential. The controller 620 (FIG. 6) can look-up the second pressure differential in the look-up table, which may be correlated to 75% filter integrity (*i.e*., a partially used filter). The controller 620 (FIG. 6) may then display this determined filter integrity on the LCD 640 (FIG. 6) such that a user can know the remaining filter integrity (filter life) of the filter 800.

With the three-way valve 1402 transitioned to the second state, the controller 620 (FIG. 6) may activate the motor 820 at a predefined speed to draw in ambient air 1422 from the ambient environment 1420 into the second inlet port 1406 and along the second flow path 1414, through the three-way valve 1402, and along the common flow path 1418 to the filter 800. As the motor 820 drives ambient air 1422 through the filter 800, the controller 620 may monitor a flow rate of the ambient air 1422 through the evacuator module 1400. For example, as the motor 820 drives ambient air 1422 through the filter 800, the controller 620 can receive flow measurements from the sensor 800b, for example, which may be indicative of the quality of the filter 800.

The controller 620 may compare the measured flow rate to a threshold flow rate. The threshold flow rate may be stored in the memory 624 (FIG. 6). Based on the measured flow rate reaching or dropping below the threshold flow rate, the controller 620 may generate an alert, such as a visual alert on the LCD 640 (FIG. 6) and/or an audio alert with the speaker 650 (FIG. 6), to inform a user that the filter 800 should be replaced. Based on the measured flow rate reaching or dropping below the threshold flow rate, the controller 620 may prevent activation of one or more modules in the modular surgical system, such as the energy module 204 or the evacuator module 1400, for example, until the filter 800 is replaced.

The memory 624 may include a look-up table that correlates measured flow rate at the predefined speed of the motor 820, discussed above, to filter integrity. For example, with a new filter 800 positioned in the evacuator module 208 and ambient air 1422 being driven through the filter 800 by the motor 820 at the predefined speed, the controller 620 (FIG. 6) may measure a first flow rate at the second sensor 820b. The controller 620 can look-up the first flow rate in the look-up table, which may be correlated to 100% filter integrity (*i.e*., new filter). The controller 620 (FIG. 6) may then display this determined filter integrity on the LCD 640 (FIG. 6) such that a user can know the remaining filter integrity (filter life) of the filter 800.

At a later time, after the filter 800 has filtered an amount of smoke 812, ambient air 1422 may be driven through the filter 800 by the motor 820 at the predefined speed and the controller 620 (FIG. 6) may measure a second flow rate less than the first flow rate. The controller 620 (FIG. 6) can look-up the second flow rate in the look-up table, which may be correlated to 75% filter integrity (*i.e.,* a partially used filter). The controller 620 (FIG. 6) may then display this determined filter integrity on the LCD 640 (FIG. 6) such that a user can know the remaining filter integrity (filter life) of the filter 800.

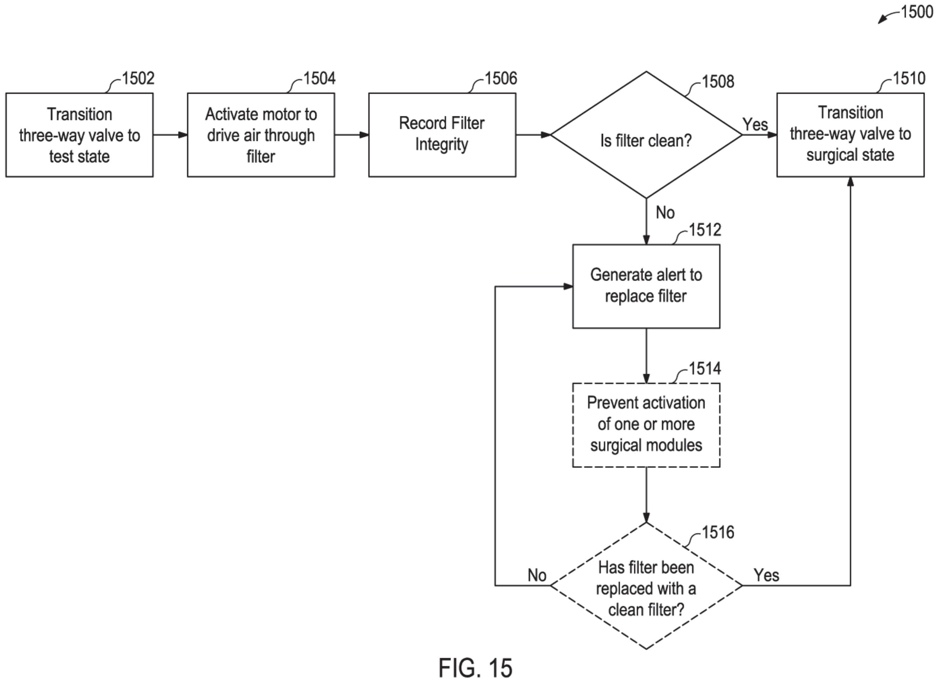

Referring now to FIG. 15, a flow diagram 1500 for operating the evacuator module 1400 of FIG. 14 is provided, according to at least one aspect of the present disclosure. In some embodiments, the flow diagram 1500 is embodied as an algorithm, stored in the memory 624 (FIG. 6) of the controller 620 (FIG. 6), and is executable by the processor 622 (FIG. 6). In some embodiments, the algorithm is executable by the processor 622 based on the evacuator module 1400 being powered on, a threshold amount of time elapsing since a surgical instrument was activated, or a user providing an input to the touchscreen 630 (FIG. 6), for example.

Example operation of the evacuator module 1400 is now provided with reference to FIG. 15, and with continued reference to FIGS. 6 and 14. At step 1502, the controller 620 transitions the three-way valve 1402 to the second (test) state. At step 1504, the controller 620 controls the motor 820 (and therefore, the pump 804) to drive (draw in) ambient air 1422 from the ambient environment 1420 into the second inlet port 1406 and along the second flow path 1414, through the three-way valve 1402, and along the common flow path 1418 to the filter 800. At step 1506, the controller 620 determines the integrity of the filter 800, such as by measuring a flow rate through the filter 800 or determining a pressure differential across the filter 800 and consulting a look-up table, as described elsewhere herein. The controller 620 may then record this determined integrity in the memory 624, for example.

At step 1508, the controller 620 determines if the filter 800 is clean. For instance, at step 1508, the controller 620 may measure the flow rate through the filter 800 using sensor 820b, or determine a pressure differential across the filter 800 using the first and second sensors 820a,b, and determine if the filter 800 is clean by comparing one or both of the measured/determined parameters to corresponding thresholds, which may be stored in the memory 624.

Based on determining that the filter 800 is still clean (e.g., the measured pressure differential is below a pressure threshold), the controller 620 may proceed to step 1510 and transition the three-way valve 1402 to the first (surgical) state, thus allowing the evacuator module 1400 to evacuate smoke 812 from a surgical site. If a determination is made that the filter 800 is not clean (e.g., the measured pressure differential has reached or exceeds a pressure threshold), the controller 620 may then proceed to step 1512 and generate an alert, such as a visual alert on the LCD 640 and/or an audio alert with the speaker 650, to inform a user that the filter 800 needs attention and should possibly be replaced. Based on providing the alert at step 1512, the controller 620 may proceed to step 1510 and transition the three-way valve 1402 back to the first (surgical) state.

In some embodiments, the algorithm 1500 optionally includes a step 1514 at which the controller 620 prevents activation of one or more surgical modules in the modular surgical system, such as the energy module 204 (FIG. 2) or the evacuator module 1400, for example, until the filter 800 is rehabilitated or replaced.

In some embodiments, the algorithm 1500 optionally includes a step 1516 at which the controller 620 determines if the filter 800 has been replaced with a clean filter. For example, at step 1516, the controller 620 can determine if the filter 800 has been replaced with a clean filter by activating the motor 820 to drive ambient air 1422 to the filter 800 and comparing the measured flow rate and/or the determined pressure differential to their corresponding threshold values. Once it is determined that the replaced filter 800 is a clean filter (e.g., the measured pressure differential is below a pressure threshold), the controller 620 may proceed to step 1510 and transition the three-way valve 1402 back to the first (surgical) state, thus allowing the evacuator module 1400 to be used to evacuate smoke 812 from a surgical site. If it is determined that the replaced filter 800 is not a clean filter (e.g., the measured pressure differential is above a pressure threshold), the controller 620 may proceed back to step 1512 and generate an alert, such as a visual alert on the LCD 640 and/or an audio alert with the speaker 650, to inform a user that the replaced filter 800 is not a clean filter and should be replaced.

The foregoing evacuator module 1400 with the three-way valve 1402 allows for the quality of the filter 800 to be determined at any desired time when a surgical instrument is not being used to generate smoke, such as during initial startup, in the middle of a surgical procedure with the surgical instrument still positioned at the surgical site, or at the conclusion of the surgical procedure. Notably, using a separate and distinct flow path that draws ambient air 1422 from the surround environment 1420 provides the benefit of removing the variability associated with a flow path that is used to draw smoke from a surgical site, which may be contaminated.

### Improved Usability for Filter Change via Procedure Indexing

Referring again to FIG. 8, and as discussed above, the evacuator module 208 can include the plurality of sensors 820a-d positioned along the flow path 806 for measuring one or more parameters associated with the smoke 812 and/or filtered air 814 flowing along the flow path 806, such as a flow rate of the smoke 812/filtered air 814 along the flow path 806.

In use, a clinician may activate a surgical instrument, such as one of surgical instruments 300, 330, 360 (FIG. 3), to cut (treat) a target tissue. Once the surgical instrument is activated, the controller 620 (FIG. 6) can control the motor 820 (and therefore, the pump 804) to drive (draw in) smoke 812 generated by the surgical instrument through the evacuator module 208, while also initiating the timer 626 (FIG. 6) to measure an amount of time (in seconds, for example) that the surgical instrument is activated. In addition, the controller 620 may communicate with the sensor(s) 820a-d to determine the flow rate of smoke 812 (measured in cm³/second or m³/second, for example) that passes to the filter 800. Utilizing the determined time of activation and the detected rate of smoke 812, the controller 620 can calculate (determine) the volume of smoke 812 (measured in cm³ or m³, for example) that has been drawn through the filter 800.

The controller 620 (FIG. 6) may also be programmed to monitor the cumulative volume of smoke 812 that passes to the filter 800 over time, which may be indicative of filter life. More specifically, the controller 620 may compare the monitored volume to a volume threshold and perform a function based on the monitored volume of smoke 812 approaching, reaching, or exceeding the volume threshold. The volume threshold may be stored in the memory 624 (FIG. 6). The function may include generating an alert, such as a visual alert on the LCD 640 (FIG. 6) and/or an audio alert with the speaker 650 (FIG. 6), to inform a user that the filter 800 should be replaced. The function may also include preventing activation of one or more modules in the modular surgical system, such as the energy module 204 (FIG. 2) or the evacuator module 208, for example, until the filter 800 is replaced.

In addition to the above, the controller 620 (FIG. 6) may further determine if the surgical instrument is being used in an open procedure or a laparoscopic procedure. An open procedure generally requires a single large incision that allows for better visibility of the surgical site, whereas a laparoscopic procedure generally requires multiple smaller cuts where a camera extends through one of these cuts to view the surgical area. During a laparoscopic procedure, the motor 820 of the evacuator module 208 generally draws smoke 812 from the surgical site at a first speed that is slower when compared to the speed of the motor 820 for an open procedure as the generated smoke 812 is contained within the patient (*i.e*., not exposed to the surrounding environment, like the operating room). During an open procedure, the motor 820 generally draws smoke 812 from the surgical site at a second speed faster than the first speed as the generated smoke 812 is exposed to the surrounding environment, like the operating room. Accordingly, the evacuator module 208 generally operates in differing manners (e.g., different speeds) depending on if it is used in laparoscopic or open procedures, which can affect the quality of the filter 800 over time.

In some embodiments, the memory 624 (FIG. 6) stores a first threshold number of uses of the filter 800 for open procedures and a second threshold number of uses of the filter 800 for laparoscopic procedures, where the second threshold number of uses may be greater than the first threshold number of uses. In other embodiments, the memory 624 stores a common threshold number of uses of the filter 800 for open and closed procedures where one open procedure is equated to multiple laparoscopic procedures. For example, as will be discussed in more detail below, in tracking the number of uses of the filter 800, the controller 620 (FIG. 6) may decrement a filter counter by one for each laparoscopic procedure, but decrement the filter counter by more than one (2, 3, or 4, for example) for each open procedure.

Referring to FIGS. 6 and 8, in example use, the controller 620 may monitor (track) the number of procedures in which the filter 800 has been used. The controller 620 can set a first counter for open procedures and set a second counter for laparoscopic procedures. The first and second counters may correspond to the first and second threshold numbers of uses of the filter 800, described above. For example, the controller 620 can set the first counter to X (i.e., X number of uses for open procedures) and the second counter to Y (i.e., Y number of uses for laparoscopic procedures).

Prior to using a surgical instrument, such as one of surgical instruments 300, 330, 360 (FIG. 3), to cut (treat) a target tissue, a user can provide an input to the touchscreen 630 of the header module 202 indicating the type of surgical procedure to be performed (open vs. laparoscopic). Based on the type of procedure to be performed, the controller 620 can decrement the associated counter. For example, the controller 620 may assign the first counter (open procedure) at a first number of uses X and the second counter (laparoscopic procedure) at a second number of uses Y. A user may provide an input to the controller 620 via the touchscreen 630, indicating that a surgical instrument is to be used in an open procedure. Accordingly, the controller 620 decrements the first count by 1 (*i.e.,* X - 1).

Based on the first or second number counters approaching or reaching zero, the controller 620 can generate an alert, such as a visual alert on the LCD 640 and/or an audio alert with the speaker 650, to inform a user that the filter 800 should soon be replaced. In some such embodiments, the controller 620 may further prevent activation of one or more modules in the modular surgical system, such as the energy module 204 or the evacuator module 208, for example, until the filter 800 is replaced.

### Real-Time Monitoring and Datalogging of Pressure Drop through a Filter

As discussed above, current methods for determining when to replace the filter are based, at least in part, on use time, which may not adequately quantify the amount of particulate that has been captured by a filter and can result in the filter being replaced too early or too late.

All filters present a resistance to airflow. Notably, a filter at the beginning of its life (*i.e.,* a new filter) will have a defined and baseline resistance to airflow. Over time, as particulates are captured by (within) the filter, this resistance correspondingly increases and may be indicative of filter life and used to better determine when the filter should be replaced.

A filter may lose its desired effectiveness when the resistance to airflow reaches a threshold resistance. This threshold resistance may be provided by the manufacturer, for instance. The threshold resistance may be provided to the controller 620 (FIG. 6) by a user via the touchscreen 630 (FIG. 6). The threshold resistance may be stored in the memory 624 (FIG. 6) and may be retrievable by the processor 622 (FIG. 6). In other embodiments, the threshold resistance may be read by the controller 620 based on the filter being installed in the evacuation module 208 (FIG. 8) or may be stored in the memory 624 (FIG. 6) and managed by the controller 620 (FIG. 6). The threshold resistance may be a threshold resistance for an open surgical procedure or a laparoscopic surgical procedure.

Referring again to FIG. 8, as discussed herein, the evacuator module 208 may include the plurality of sensors 820a-d positioned along the flow path 806 for measuring one or more parameters associated with the smoke 812 and/or filtered air 814, such as a flow rate of the smoke 812/filtered air 814 along the flow path 806 and/or a pressure differential across the filter 800 using the first and second sensors 820a,b, for example.

When the evacuator module 208 is in a stacked configuration with the header module 202, such as in the configuration shown in FIG. 6, for example, the plurality of sensors 820a-d can be in operable communication with the controller 620 (FIG. 6), such as along the data interface 610 (FIG. 6), and can receive power from the header module 202 (FIG. 6) via the power interface 608 (FIG. 6). The controller 620 (FIG. 6) may receive measurements obtained from the sensors 820a-d, determine the resistance of the filter 800, and compare this determined resistance to the threshold resistance of the filter 800. For instance, the resistance of the filter 800 may be defined as the pressure differential across the filter 800, which may be measured by the controller 620 via the first sensor 820a and the second sensor 820b. In some instances, the resistance may be defined as a difference between the ambient room conditions (pressure) and post filter flow for open cases. In some instances, the resistance may be defined as the difference between pre-filter flow and post-filter flow for laparoscopic cases. Based on the determined resistance approaching, reaching, or exceeding the threshold resistance, the controller 620 may generate an alert to inform a user that the filter 800 should be replaced and/or prevent activation of one or more modules in the modular surgical system, such as the energy module 204 (FIG. 2) or the evacuator module 208, for example, until the filter 800 is replaced. The alert may include a visual alert via the LCD 640 (FIG. 6) and/or an audio alert via the speaker 650 (FIG. 6).

Based on the determined resistance approaching, reaching, or exceeding the threshold resistance, the controller 620 (FIG. 6) may generate an alert to inform a user that the filter 800 has a finite amount of life (tracked number of uses) remaining. In such embodiments, the generated alert can inform the user that the evacuator module 208 may still be used, such as to complete a given surgical procedure, but that the filter 800 should be replaced soon, such as at the conclusion of the given procedure.

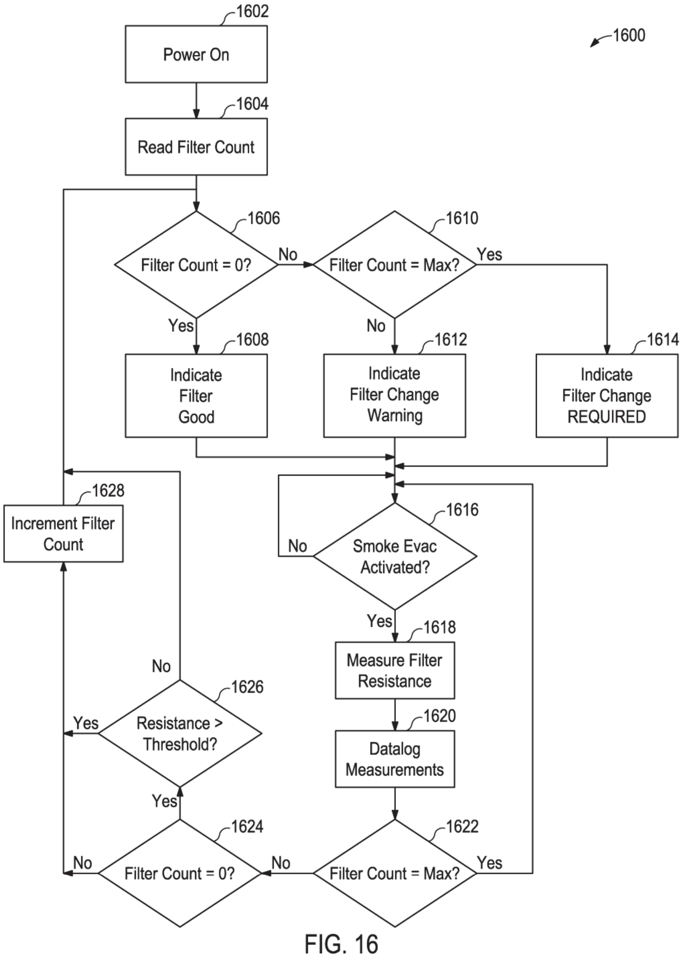

Referring now to FIG. 16, illustrated is an example flow diagram 1600 for measuring filter life, according to at least one aspect of the present disclosure. In some embodiments, the flow diagram 1600 is embodied as an algorithm, stored in the memory 624 (FIG. 6) of the controller 620 (FIG. 6), and is executable by the processor 622 (FIG. 6).

Discussion of the flow diagram 1600 is now provided, with continued reference to FIGS. 6 and 8. At step 1602, the evacuator module 208 is powered on, such as by placing the evacuator module 208 into a stacked configuration with the header module 202, as shown in FIG. 6 for example. The controller 620 may then proceed to step 1604 at which the controller 620 can read (retrieve) a filter count. In some embodiments, the filter count is a value useable by the controller 620 to track the number of uses remaining for a filter once the resistance of the filter has reached or exceeded the threshold resistance, as discussed above. In some embodiments, the filter 208 is assigned a filter count of 0 based on being installed into the evacuator module 208. In other embodiments, the filter count is provided to the controller 620 by a user via the touchscreen 630. The filter count may be stored in the memory 624.

The controller 620 may then proceed to step 1606 at which the controller 620 determines if the filter count is equal to 0. Based on the controller 620 determining that the filter count is equal to 0, the controller 620 may proceed to step 1608 and generate an indication, such as a visual indication on the LCD 640 and/or an audio indication with the speaker 650, to inform a user that the filter 800 is a good filter (*i.e.,* does not have a tracked number of uses remaining).

If the controller 620 determines that the filter count is not equal to 0 at step 1606, the controller 620 may proceed to step 1610 and determine if the filter count is equal to a maximum filter count. The maximum filter count may correspond to the number of uses remaining for a filter once the resistance of the filter has reached or exceeded the threshold resistance. The maximum filter count may be provided by the manufacturer, and may be provided to the controller 620 via the touchscreen 630 and stored in the memory 624.

Based on the controller 620 determining that the filter count is not equal to the maximum filter count, the controller 620 may proceed to step 1612 and generate an indication, such as a visual indication on the LCD 640 and/or an audio indication with the speaker 650, to inform a user that the filter 800 has a tracked (finite) number of uses remaining and will soon need replaced. In some embodiments, the algorithm 1600 further includes a step at which, based on the controller 620 determining that the filter count is less than the maximum filter count, the controller 620 determines a difference between the filter count and the maximum filter count (*i.e.,* the number of uses remaining of the filter 800) and displays the determined difference on the LCD 640.

If the controller 620 determines that the filter count is equal to the maximum filter count, the controller 620 may proceed to step 1614 and generate an alert, such as a visual alert on the LCD 640 and/or an audio alert with the speaker 650, to inform a user that the filter 800 should be replaced. The algorithm 1600 may further include a step at which, based on the controller 620 determining that the filter count is equal to the maximum filter count, the controller 620 prevents activation of one or more modules in the modular surgical system, such as the energy module 204 (FIG. 2) or the evacuator module 208, for example, or may prevent advancement to the next step of the algorithm (step 1616) until the filter 800 is replaced.

From steps 1608, 1612, and 1614, the controller 620 may proceed to step 1616 at which the controller 620 determines if the evacuator module 208 has been activated. The controller 620 may determine if the evacuator module 208 is activated by detecting if power is being supplied to the motor 820 via the power interface 608, such as with the current sensor 628, or if the controller 620 is receiving measurements from the sensors 820a-d. If it is determined that the evacuator module 208 is not activated, the controller 620 loops at step 1616 until the controller 620 determines that the evacuator module 620 has been activated. In some embodiments, the controller 620 may terminate the algorithm 1600 based on a threshold amount of time elapsing at step 1616. The threshold amount of time may be stored in the memory 624 and the elapsed time may be measured by the timer 626.

If it is determined that the evacuator module 208 is activated, the controller 620 may proceed to step 1618 to measure filter resistance, such as using measurements obtained by the sensors 820a-d, as discussed elsewhere herein. The controller 620 may then proceed to step 1620 and datalog (record) the measured resistance in the memory 624.

The controller 620 may then proceed to step 1622 to compare the filter count to the maximum filter count. Based on the controller 620 determining that the filter count is equal to the maximum filter count, the controller 620 may proceed back to step 1616 and again determine if the evacuator module 208 is activated. Alternatively, if the controller 620 determines that the filter count is equal to the maximum filter count at step 1622, the controller 620 may generate an alert, such as a visual alert on the LCD 640 and/or an audio alert with the speaker 650, to inform a user that the filter 800 had reached its maximum filter life and should be replaced. If the controller 620 determines that the filter count is equal to the maximum filter count at step 1622, the controller 620 may prevent activation of one or more modules in the modular surgical system, such as the energy module 204 (FIG. 2) or the evacuator module 208, for example, until the filter 800 is replaced. In alternative embodiments, step 1622 is omitted and the controller 620 proceeds from step 1620 to step 1624.

If the controller 620 determines that the filter count is not equal to the maximum filter count, the controller 620 may proceed to step 1624 and determine if the filter count is equal to 0. Based on the controller 620 determining that the filter count is equal to 0, which is indicative of a "good" quality filter that does not have a tracked number of uses remaining, the controller 620 may proceed to step 1626 and compare the resistance of the filter to a resistance threshold, as discussed elsewhere herein.

Based on the controller 620 determining that the resistance is less than the resistance threshold, the controller 620 may proceed back to step 1606. Based on the controller 620 determining that the resistance is greater than the resistance threshold, the controller 620 may proceed to step 1628 and increment the filter count, and transition the filter from a good state, at which the filter 800 does not have a tracked number of uses remaining, to a finite state, at which the filter has a tracked (finite) number of uses remaining. For example, the controller 620 may determine the resistance for a "good" filter (filter count of 0; in a "good" state) exceeds a resistance threshold. Based on the determination, the controller 620 may proceed to step 1628 and increment the filter count from 0 to 1, transitioning the filter to the finite state.

At step 1624, based on the controller 620 determining that the filter count is not equal to 0, which is indicative of a filter with only a tracked (finite) number of uses remaining, the controller 620 may proceed to step 1628 and increment the filter count closer to (or to) the maximum filter count.

The foregoing algorithm 1600 allows the controller 620 to dynamically track the quality of a filter and assign a threshold number of remaining uses once a measured resistance reaching or exceeding a threshold resistance, thereby improving the confidence of the controller 620 of knowing when the filter 800 should be replaced.

In some embodiments, the controller 620 (FIG. 6) may account for a combination of filter time use and flow resistance, which may increase the maximum life of the filter. As one example, a surgical room may only have light smoke cases, yielding a minimal change in resistance over time. However, the life span of the filter may run out based on an elapsed amount of activation time reaching a time threshold. As another example, a cancer hospital may generate large amounts of smoke, causing the filter to reach a predetermined resistance limit while not achieving the activation time limit minimum requirement. Accordingly, the controller 620 (FIG. 6) may monitor filter time and resistance over time and may determine life based on a combination of both to extend the life of the filter.

### Smoke Evacuator Filter Features to Prevent Reusage

In some instances, a filter may have reached a point where it has lost its desired or operable effectiveness. Accordingly, a controller may alert a user that the filter should be replaced. Despite this alert, a user may (willingly or unknowingly) attempt to use the filter past its effective operability, which may not adequately filter smoke generated by a surgical instrument. Accordingly, it may be desirable to include a secondary system for preventing use of a filter that has reached its end-of-life or end of its useful life.

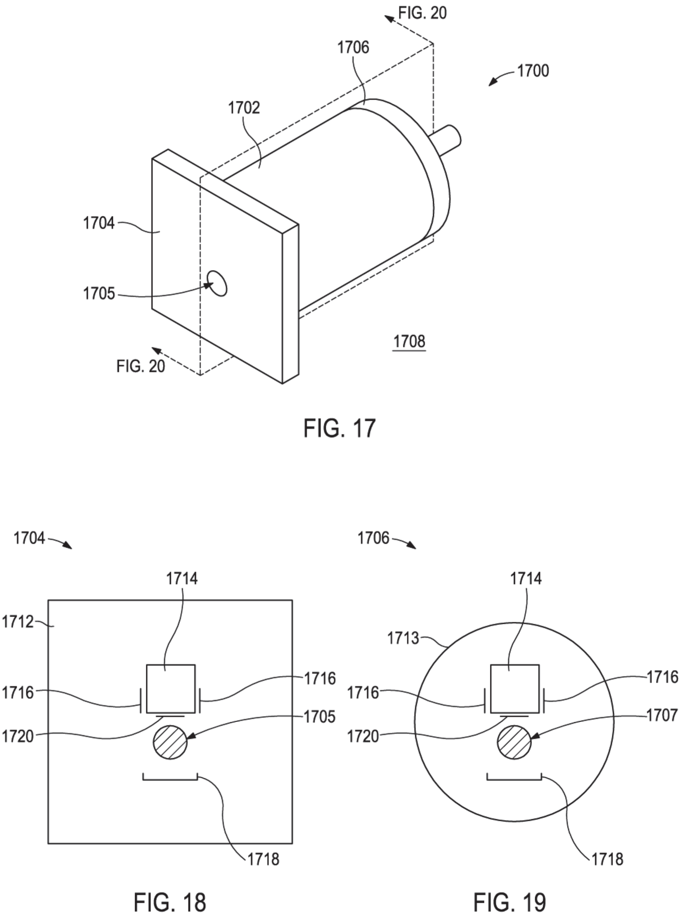

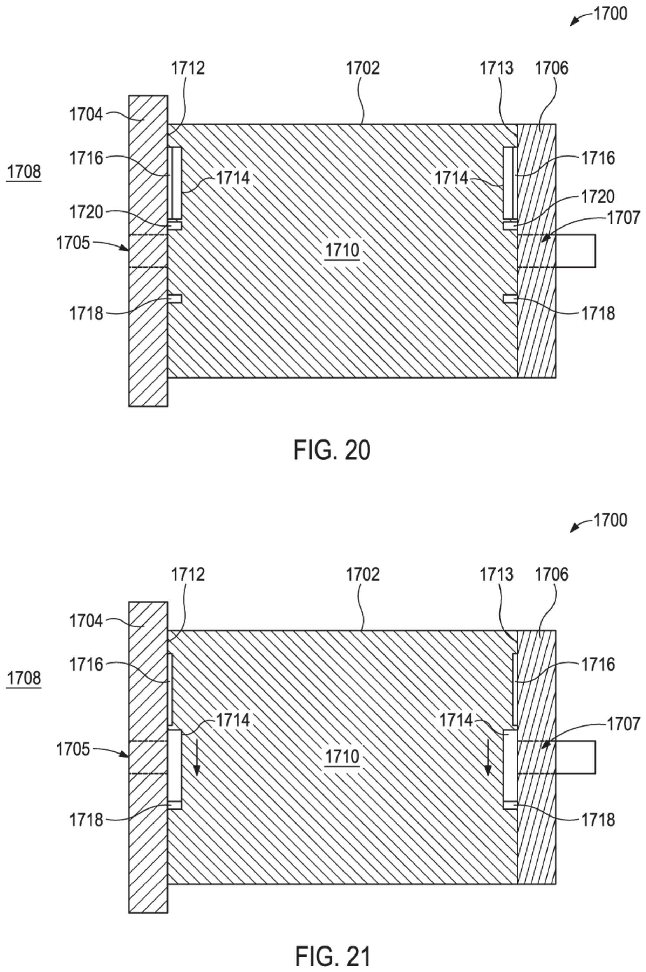

FIG. 17 is a schematic diagram of an example filter 1700, according to at least one aspect of the present disclosure. The filter 1700 may be the same as or similar to the filter 800 (FIG. 8). As illustrated, the filter 1700 includes a filter body 1702, an inlet (front) layer 1704 extending from (arranged at) a first side of the filter body 1702, and an outlet (back) layer 1706 extending from (arranged at) a second side of the filter body 1702 opposite the first side of the filter body 1702. The inlet layer 1704 defines an inlet aperture 1705 that facilitates fluid communication between an exterior environment 1708 and an interior 1710 (see FIGS. 20 and 21) of the filter body 1708. Similarly, the outlet layer 1706 defines an outlet aperture 1707 (see FIGS. 19-21) facilitates fluid communication between the exterior environment 1708 and the interior 1710. The inlet aperture 1705 enables smoke, such as smoke 812 (FIG. 8), to enter the filter 1700 to be filtered. The outlet aperture 1707 enables filtered smoke, such as the filtered air 814 (FIG. 8) to exit the filter 1700.

FIG. 18 illustrates an interior surface 1712 of the inlet layer 1704, and FIG. 19 illustrates an interior surface 1713 of the outlet layer 1706, according to at least one aspect of the present disclosure. As illustrated, the filter 1700 further includes plates 1714 movable along the interior surfaces 1712, 1713 of the inlet and outlet layers 1704, 1706, respectively, and between a first or "stowed" state (as shown in FIGS. 18-20) and a second or "deployed" state (as shown in FIG. 21). In the stowed state, the plates 1714 are displaced from the inlet and outlet apertures 1705, 1707 to enable smoke to enter the filter body 1702 from the exterior environment 1708, via the inlet aperture 1705, and exit the filter body 1702 as filtered air, via the outlet aperture 1707. In contrast, in the deployed state, the plates 1714 overlap (cover, occlude, etc.) the inlet and outlet apertures 1705, 1707 preventing smoke from entering and exiting the filter body 1702.

In some embodiments, the filter 1700 further includes tracks 1716 coupled (mounted) to the interior surfaces 1712, 1713 that function to guide the plates 1714 from the stowed state to the deployed state. The filter 1700 further includes stops 1718 that function to "catch" the plates 1714 as the plates 1714 are dropped (transitioned) into their deployed states, as will be discussed in more detail below. The plates 1714 may be made of plastic, but could alternatively be made of other rigid materials, such as a metal or a composite material.

The filter 1700 further includes actuators 1720 that are movable (actuatable) between an unactuated state and an actuated state. In the unactuated state, the actuators 1720 abut (contact) the plates 1714 to maintain the plates 1714 in their stowed states. In the actuated state, the actuators 1720 are displaced from the plates 1714 to allow the plates 1714 to drop (by gravity) down to the stops 1718 to their deployed states. In some embodiments, the actuators 1720 comprise levers that are pivotably coupled to the interior surfaces 1712, 1713 and that are rotatable between the unactuated state and the actuated state. In some embodiments, the actuators 1720 comprise bars that are translatable along the interior surfaces 1712, 1713 between the unactuated state and the actuated state, such as along tracks similar to tracks 1716. In some embodiments, the actuators 1720 further comprise a motor operable to actively move the bars between the unactuated state and the actuated state. In some embodiments, the actuators 1720 further comprise a coil that is operable to generate a magnetic field to move the bars between the unactuated state and the actuated state.

In use, the filter 1700 may be operably positioned within an evacuator module, such as any evacuator module discussed herein, like evacuator module 208 (FIG. 8) or evacuator module 1400 (FIG. 14), which may be in a stacked configuration with a header module 202, such as in the configuration shown in FIG. 6. In the stacked configuration, the controller 620 (FIG. 6) of the header module may communicate with the actuators 1720, such as along the data interface 610 (FIG. 6), to transition the actuators 1720 between the unactuated and actuated states. In the stacked configuration, the controller 620 may also provide power to the the actuators 1720, such as along the power interface 608 (FIG. 6)

Example operation of the filter 1700 is now provided with reference to FIGS. 6, 8 and 17-22. A clinician may activate a surgical instrument, such as one of surgical instruments 300, 330, 360 (FIG. 3), to cut (treat) a target tissue. Based on the surgical instrument being activated, the controller 620 can control the motor 820 (and therefore, the pump 804) to drive (draw in) smoke 812 generated by the surgical instrument through the filter 1700. As the surgical instrument begins to generate smoke 812, the actuators 1700 may be in their unactuated states to hold the plates 1714 in their stowed states to allow the smoke 812 to enter the filter 1700.

At some later time, the controller 620 may determine that the filter 1700 needs to be replaced, as discussed elsewhere herein. Based on such a determination, the controller 620 may generate an alert, such as a visual alert on the LCD 640 and/or an audio alert with the speaker 650, to inform a user that the filter 1700 should be replaced. In addition, or in the alternative thereto, the controller 620 may cause the actuators 1720 to transition to the actuated state, thereby causing the plates 1714 to transition from the stowed state (FIG. 20) to the deployed state (FIG. 21), and thereby preventing additional smoke 812 from entering the filter 1700.

Accordingly, the filter 1700 provides a secondary, mechanical system that prevents a user from willing or unknowingly using the filter 1700 once it has reached the point of needing to be replaced. In some embodiments, the filter 1700 may not include the plate 1714, catch 1716, tracks 1718, and actuator 1720 on the interior surface 1713 of the outlet layer 1706.

### Smoke Evacuation Filter Weight Monitoring

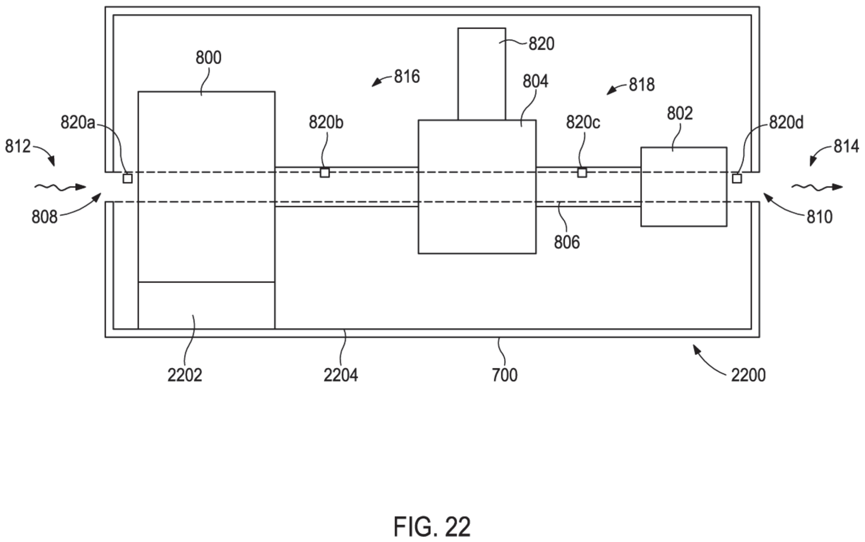

FIG. 22 depicts an example evacuator module 2200, according to at least one aspect of the present disclosure. The evacuator module 2200 may be similar in some respects to the evacuator module 208 of (FIG. 2) and therefore may be best understood with reference thereto, where like numbers used in the figures correspond to similar components not described again in detail.

Over time, the physical weight of the filter 800 may increase as particulates from the smoke 812 (mass) is built up (accumulates) on the filter 800. Accordingly, the weight of the filter 800 may be indicative of filter life. In the illustrated embodiment, the evacuator module 2200 includes a scale 2202 that may be used to measure the weight of the filter 800 over time. In some embodiments, the scale 2202 is mounted to a base 2204 of the housing 700 and the filter 800 is operably positioned such that the filter 800 sits (rests) upon the scale 2202.

When the evacuator module 2200 is in a stacked configuration with a header module 202, such as in the configuration shown in FIG. 6, for example, the scale 2202 can communicate with the controller 620 (FIG. 6), such as along the data interface 610 (FIG. 6), and can receive power from the header module 202 (FIG. 6) via the power interface 608 (FIG. 6). The controller 620 may receive the measured weight from the scale 2202 and monitor the weight of the filter 800 continuously or periodically (e.g., after a predefined amount of time has elapsed), based on the evacuator module 2200 being powered on, or in response to a user input at the touchscreen 630 (FIG. 6), as examples.

The controller 620 (FIG. 6) may compare the real-time measured weight to a threshold weight, which may be stored in the memory 624 (FIG. 6). Based on the measured weight approaching, reaching, or exceeding the threshold weight, the controller 620 (FIG. 6) may generate an alert, such as a visual alert via the LCD 640 (FIG. 6) or an audio alert via the speaker 650 (FIG. 6), to inform a user that the filter 800 should be replaced, or may prevent activation of one or more modules in the modular surgical system, such as the energy module 204 (FIG. 2) or the evacuator module 2200, for example, until the filter 800 is replaced.

Accordingly, measurements from the scale 2202 provides the controller 620 with an additional data point that can be used to determine when the filter 800 should be replaced. In addition, use of the scale 2202 prevents inadvertent reuse of a filter that has exceeded its useful life since the scale 2202 will measure a weight that is above the weight threshold.

### Automatic and Diagnostic Smoke Evacuation

Referring again to FIG. 9, as mentioned above, the evacuator module 208 may include the first air quality sensor 900 for measuring particulates that enter the evacuator module 208 and pass to the filter 800. When the evacuator module 208 is in a stacked configuration with a header module 202, such as in the configuration shown in FIG. 6, for example, the air quality sensor 900 can communicate with the controller 620 (FIG. 6) of the header module 202 (FIG. 6), such as along the data interface 610 (FIG. 6), and can receive power from the header module 202 (FIG. 6) via the power interface 608 (FIG. 6). The controller 620 may receive measurements from the air quality sensor 900 and control various operations of the modular surgical system, such as a speed of the motor 820, based on the received measurements.

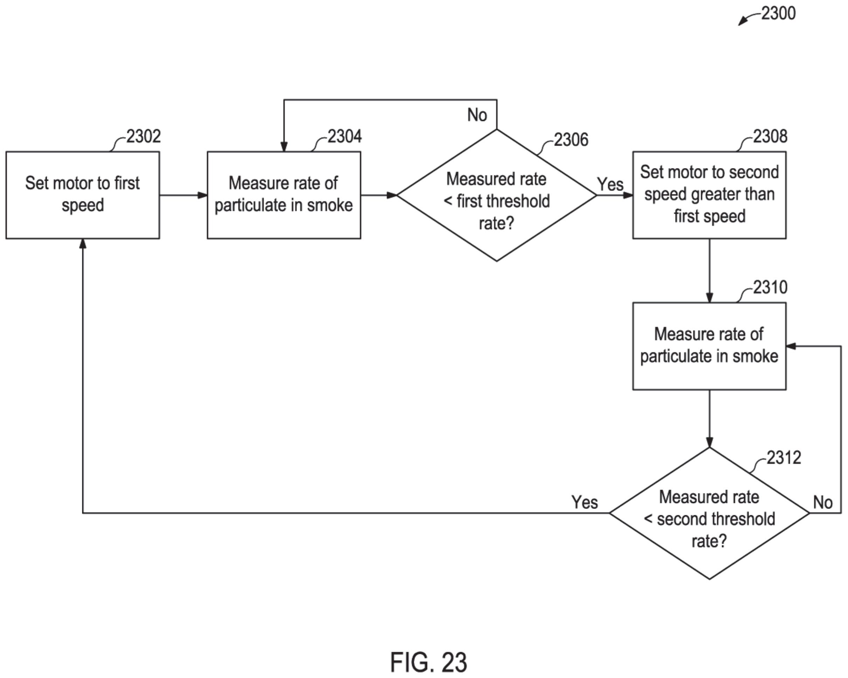

FIG. 23 depicts a schematic flow diagram 2300 for dynamically controlling a speed of a motor of the evacuation module 208, according to at least one aspect of the present disclosure. In some embodiments, the flow diagram 2300 is embodied as an algorithm, stored in the memory 624 (FIG. 6) of the controller 620 (FIG. 6), and is executable by the processor 622 (FIG. 6). In some embodiments, the algorithm is executable by the processor 622 based on the motor being actuated by the controller 620, such as in response to a surgical instrument being activated to cut tissue.

Example operation of the evacuation module 208 is now provided with reference to FIG. 23, and continued reference to FIGS. 6 and 9. A user may activate a surgical instrument, such as one of surgical instruments 300, 330, 360 (FIG. 3), to cut (treat) a target tissue. Based on the surgical instrument being activated, the controller 620 can execute the algorithm 2300, beginning at step 2302. At step 2302, the controller 620 sets the motor 820 at a first or "diagnostic" speed to drive smoke 812 generated by the surgical instrument through the evacuator module 208. The first speed may be a percentage of the maximum speed of the motor 820, such as 5%, 10%, 15%, or 20%, as examples.

The controller 620 may then proceed to step 2304 and measure the volumetric flow rate of particulate passing to the filter 800. For example, as discussed elsewhere herein, the controller 620 can monitor the one or more sensor(s) 820a-d and the air quality sensor 900 to determine the volumetric flow rate of particulate passing to the filter 800.

The controller 620 may then proceed to step 2306 and compare the measured volumetric flow rate of particulate to a first threshold volumetric flow rate, which may be stored in the memory 624. If the measured volumetric flow rate is less than the first threshold volumetric flow rate, the controller 620 may proceed back to step 2304 to measure the volumetric flow rate of particulate passing to the filter 800. If the measured volumetric flow rate is greater than the first threshold volumetric flow rate, the controller 620 may proceed to step 2308 and transition (set) the motor 820 to a second or "surgical" speed faster than the first (diagnostic) speed. The second speed may be the maximum speed of the motor 820, or may be a percentage of the maximum speed of the motor 820, such as 75%, 80%, 90%, or 95%, as examples.

The controller 620 may then proceed to step 2310 and measure the volumetric flow rate of particulate passing to the filter 800. For example, as discussed elsewhere herein, the controller 620 can monitor the sensor(s) 820a-d and the air quality sensor 900 to determine the volumetric flow rate of particulate passing to the filter 800.

The controller 620 may then proceed to step 2312 and compare the measured volumetric flow rate of particulate to a second threshold volumetric flow rate, which may be stored in the memory 624. In some embodiments, the second threshold volumetric flow rate is different (less) than the first threshold volumetric flow rate. Based on the measured volumetric flow rate being less the second threshold volumetric flow rate, the controller 620 may proceed back to step 2302 and transition (set) the motor 820 back at the first (diagnostic) speed. Based on the measured volumetric flow rate being greater the second threshold volumetric flow rate, the controller 620 may proceed back to step 2310 and measure the volumetric flow rate of particulate passing to the filter 800.

Accordingly, the algorithm 2300 enables the controller 620 to set the motor 620 to a slower, diagnostic speed, which may be slow enough as to not generate noise in the operating room, but sufficiently fast enough to detect particulates that may be generated while using a surgical instrument. Based on the detection of particulates, the controller 620 can automatically increase the speed of the motor 820 to filter unwanted particulate from the surgical site until a sufficient amount of particulate has been removed therefrom, at which point the controller 820 can automatically transition the motor 820 back to the diagnostic speed.

### Base Lining Algorithm for Smoke Evacuation / Insufflation

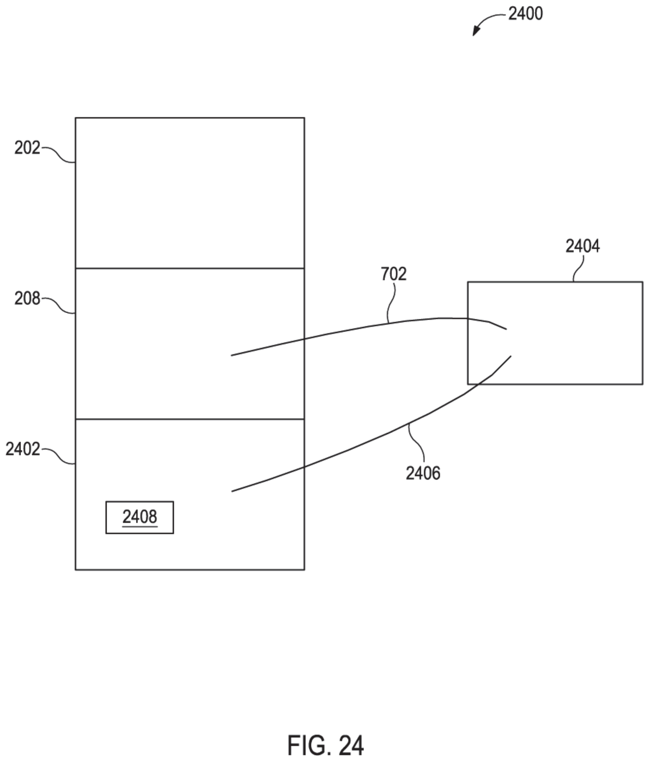

FIG. 24 depicts a schematic diagram of an example modular surgical system 2400, according to at least one aspect of the present disclosure. The modular surgical system 2400 includes the header module 202, the evacuator module 208, and an insufflation module 2402. The insufflation module 2402 may be fluidically coupled to a patient 2404 via an insufflation tube 2406 to pressurize a body cavity of the patient 2404 with a gas, such as carbon dioxide. The evacuator module 208 may also be fluidically coupled to the patient 2404 via the suction conduit 702 to draw smoke from a surgical site in the body cavity, as discussed elsewhere herein. Accordingly, a flow path may be defined from the insufflation module 2402 to the evacuator module 208 via the insufflation tube 2404, the patient 2404, and the suction conduit 702. The header module 202 may be in operable communication with the evacuator module 208 and the insufflation module 2402, such as along the data interface 610 (FIG. 6), and may provide power to the evacuator module 208 and the insufflation module 2402, such as along the power interface 608 (FIG. 6).

According to embodiments of the present disclosure, the controller 620 (FIG. 6) of the header module 202 may initiate a diagnostic procedure to detect nominal readings of the modular surgical system 2400. The controller 620 may initiate the diagnostic procedure based on the modular surgical system 2400 being placed into a stacked configuration, as shown in FIG. 24, or based on a user input provided to the touchscreen 630 (FIG. 6). The controller 620 may initiate the diagnostic procedure prior to a surgical instrument being used to cut (treat) tissue.

The nominal readings may include a pressure drop measured over a period of time, and the measured pressure drop may be indicative of a nominal leak rating along the flow path. The controller 620 may measure the pressure drop using the one or more of the sensors 820a-d (FIG. 8) of the evacuator module 208 or alternatively using a dedicated pressure sensor 2408 of the insufflation module 2402.

The nominal readings may include resistance to airflow from the insufflation module 2402 and/or to the evacuator module 208. The controller 620 may measure the resistance by measuring a current draw of the motor 820 (FIG. 8) using the current sensor 628 (FIG. 6) for a given voltage.

Based on the nominal readings, the controller 620 can adjust the insufflation module 2402, such as the pressure provided to the patient cavity via the insufflation tube 2606, for example, to allow the insufflation module 2402 to more accurately maintain a desired insufflation pressure or flow during a surgical procedure, even when the evacuator module 208 is being used.

### Utilizing Motor Resistance as a Method for Determining Filter Life

Referring again to FIGS. 6 and 8, as discussed above, when the evacuator module 208 is in a stacked configuration with the header module 202, such as in the configuration shown in FIG. 6, the sensors 820a-d can communicate with the controller 620, such as along the data interface 610, and can receive power from the header module 202 via the power interface 608. The controller 620 may receive measurements from the sensors 820a-d and control various operations of the modular surgical system based on these measurements. For example, using the sensors 820a-d, the controller 620 may measure a flow rate of the smoke 812/ filtered air 814 through the evacuator module 208 and/or pressure differential across the filter 800, which can be used to determine when the filter 800 should be replaced.

The controller 620 may also measure the amount of current supplied to the motor 820 using the current sensor 628, which may be indicative of the remaining life of the filter 800. When a new filter 800 is installed in the evacuator module 208, for instance, the controller 620 may provide current to the motor 820 along the power interface 608 to achieve a predetermined "new-filter" flow rate through the filter 800, and the predetermined new-filter flow rate may be stored in the memory 624. The controller 620 may achieve the predetermined new-filter flow rate by monitoring data received from flow sensors 820a-d and adjusting the amount of current supplied to the motor 820 until the predetermined new-filter flow rate is achieved. Once the predetermined new-filter flow rate is achieved, the controller 620 may measure the amount of current required to achieve the predetermined new-filter flow rate ("baseline current") via the current sensor 628. The controller 620 may store the baseline current in the memory 624.

As the filter 800 accumulates particulate over time, the amount of current supplied to the motor 820 may need to increase in order to achieve a predetermined surgical flow rate through the motor 820. The predetermined surgical flow rate may be stored in the memory 624. As the controller 620 provides power to the motor 820 to achieve the predetermined surgical flow rate, the controller 620 may compare the real-time current measured by the current sensor 628 to a current threshold, which may be a predefined amount greater than the baseline current.

In some embodiments, based on the measured real-time current approaching, reaching, or exceeding the current threshold, the controller 620 may generate an alert, such as a visual alert via the LCD 640 or an audio alert via the speaker 650, to inform a user that the filter 800 should be replaced, or may prevent activation of one or more modules in the modular surgical system, such as the energy module 204 or the evacuator module 208, for example, until the filter 800 is replaced.

In other embodiments, or in addition thereto, the controller 620 may be programmed to set a count for a new filter and decrement the count based on measurements obtained from the current sensor 628. For example, when a new filter 620 is installed in the evacuator module 208, the controller 620 may assign the filter to have a predefined number of life units ("LUs"), such as 25, 50, 75, or 100 LUs, for example. The predefined number of LUs may be stored in the memory 624. In use, the controller 620 may measure the current supplied to the motor 820 periodically, such as every 5, 10, or 15 minutes, or continuously. Based on the measured current increasing by an amount Y, the controller 620 can decrement the stored LUs a corresponding amount or magnitude; *i*.*e*., Remaining LUs = Stored LUs - (measured current - baseline current).

The remaining LUs may be stored in the memory 624 such that subsequent uses of the filter 800 begin with the remaining LUs (*i.e.,* the LUs are not reset to the predefined number of LUs at the conclusion of each procedure). Once the remaining LUs reach or near 0, the controller 620 may generate an alert, such as a visual alert via the LCD 640 or an audio alert via the speaker 650, to inform a user that the filter 800 should be replaced, or may prevent activation of one or more modules in the modular surgical system, such as the energy module 204 (FIG. 2) or the evacuator module 208, for example, until the filter 800 is replaced. In some embodiments, the controller 620 may require a minimum number of LUs in order to activate the motor 820. In some embodiments, the controller 620 may decrement the LUs by a predefined amount, such as 1, 2, or 3, for example, at the outset or conclusion of each use of the filter 800 to ensure than expiration dates are enforced.

Embodiments disclosed herein include:
A. A system comprising an evacuator module that provides a housing that defines an inlet port and an outlet port, a flow path extending between the inlet and outlet ports, a pump drivable by a motor to draw smoke into the inlet port and along the flow path, and an air quality sensor positioned at a location along the flow path to measure a parameter. The system further comprises a controller in operable communication with the air quality sensor and the motor, wherein the controller is operable to control the motor based on the parameter measured by the air quality sensor.
B. A system comprising an evacuator module that provides a housing, a first inlet port defined in the housing and facilitating fluid communication with a surgical site, a second inlet port defined in the housing and facilitating fluid communication with an ambient environment, a three-way valve arranged within the housing and including a first inlet in fluid communication with the first inlet port via a first flow path, a second inlet in fluid communication with the second inlet port via a second flow path, and an outlet. The system further comprises a pump drivable by a motor and in fluid communication with the outlet via a third flow path.
C. A system comprising an evacuator module that provides a housing that defines an inlet port and an outlet port, a flow path extending between the inlet and outlet ports, a filter arranged in the flow path, a pump drivable by a motor to draw smoke into the inlet port and through the filter, a flow sensor arranged to sense a flow rate of the smoke conveyed to the filter, and an air quality sensor arranged to measure an amount of particulate in the smoke. The system further provides a controller in operable communication with the air quality sensor, the flow sensor, and the motor, wherein the controller is operable to set the motor to a first speed, determine a volumetric flow rate of particulate conveyed to the filter based on the amount of particulate in the smoke and the flow rate of smoke conveyed to the filter, compare the volumetric flow rate of particulate conveyed to the filter to a threshold volumetric flow rate, and decrease the first speed to a second speed based on the volumetric flow rate of particulate conveyed to the filter being less than the threshold volumetric flow rate.

Each of embodiments A-C may have one or more of the following additional elements in any combination: Element 1: wherein the air quality sensor comprises a volatile organic compound (VOC) sensor. Element 2: wherein the evacuator module further includes a filter arranged in the flow path, and the air quality sensor is positioned upstream of the filter. Element 3: wherein the evacuator module further includes a flow sensor in communication with the controller and arranged to sense a flow rate of the smoke conveyed to the filter, wherein the parameter comprises an amount of particulate in the smoke, and wherein the controller is further operable to determine a volumetric flow rate of particulate conveyed to the filter based on the amount of particulate in the smoke and the flow rate of smoke conveyed to the filter. Element 4: wherein the controller is further operable to set the motor to a first speed, compare the volumetric flow rate of particulate conveyed to the filter to a first threshold volumetric flow rate, and decrease the first speed to a second speed based on the volumetric flow rate of particulate conveyed to the filter being less than the first threshold volumetric flow rate. Element 5: wherein the controller is further operable to compare the volumetric flow rate of particulate conveyed to the filter to a second threshold volumetric flow rate based on the volumetric flow rate of particulate conveyed to the filter being greater than the first threshold volumetric flow rate and set the motor to a third speed greater than the first speed based on the volumetric flow rate of particulate conveyed to the filter being greater than the second threshold volumetric flow rate. Element 6: wherein the second threshold volumetric flow rate is greater than the first threshold volumetric flow rate. Element 7: wherein the controller is further operable to maintain the first speed based on the volumetric flow rate of particulate conveyed to the filter being less than the second threshold volumetric flow rate. Element 8: wherein the evacuator module further includes a filter arranged in the flow path, and the air quality sensor is positioned downstream of the filter. Element 9: wherein the evacuator module further includes a flow sensor in communication with the controller and arranged to sense a flow rate of filtered air discharged from the filter, wherein the parameter comprises an amount of particulate in the filtered air, and wherein the controller is further operable to determine a volumetric flow rate of particulate in the filtered air based on the amount of particulate in the filtered air and the flow rate of the filtered air discharged from the filter. Element 10: wherein the controller is further operable to compare the volumetric flow rate of particulate in the filtered air to a threshold volumetric flow rate and prevent the motor from driving the pump based on the volumetric flow rate exceeding the threshold volumetric flow rate. Element 11: wherein the controller is further operable to determine an effectiveness of the filter. Element 12: wherein the three-way valve is transitionable between a first state, in which the surgical site is fluidically coupled to the pump via the first and third flow paths, and the ambient environment is fluidically decoupled from the pump and a second state, in which the ambient environment is fluidically coupled to the pump via the second and third flow paths, and the surgical site is fluidically decoupled from the pump. Element 13: further comprising a controller operable to transition the three-way valve between the first and second states. Element 14: wherein the controller is in operable communication with the motor and further operable to transition the three-way valve to the first state to allow the pump to draw smoke from the surgical site and generated by a surgical instrument transition the three-way valve to the second state to allow the pump to draw ambient air from the ambient environment based on the surgical instrument ceases to generate smoke. Element 15: wherein the evacuator module further includes a filter arranged in the third flow path, and the controller is further operable to determine a status of the filter based on the pump drawing ambient air from the ambient environment. Element 16: wherein the evacuator module further includes a first pressure sensor arranged upstream of the filter to measure a first pressure and a second pressure sensor arranged downstream of the filter to measure a second pressure, wherein the controller determines a pressure differential across the filter based on the first and second pressures, and wherein the status of the filter is determined by comparing the pressure differential to a threshold pressure differential. Element 17: wherein the controller prevents the motor from driving the pump based on the pressure differential exceeding the threshold pressure differential.

By way of non-limiting example, exemplary combinations applicable to A, B, and C include: Element 1 with Element 2; Element 2 with Element 3; Element 2 with Elements 3 and 4; Element 2 with Elements 3-5; Element 2 with Elements 3-6; Element 2 with Elements 3-5 and Element 7; Element 8 with Element 9; Element 8 with Elements 9 and 10; Element 8 with Elements 9-11; Element 12 with Element 13; Element 12 with Elements 13 and 14; Element 12 with Elements 13-15; Element 12 with Elements 13-16; Element 12 with Elements 13-17.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The use of directional terms such as above, below, upper, lower, upward, downward, left, right, and the like are used in relation to the illustrative embodiments as they are depicted in the figures, the upward direction being toward the top of the corresponding figure and the downward direction being toward the bottom of the corresponding figure.

## Claims

1. A system, comprising:
an evacuator module that provides:
a housing that defines an inlet port and an outlet port;
a flow path extending between the inlet and outlet ports;
a pump drivable by a motor to draw smoke into the inlet port and along the flow path; and
an air quality sensor positioned at a location along the flow path to measure a parameter; and
a controller in operable communication with the air quality sensor and the motor, wherein the controller is operable to control the motor based on the parameter measured by the air quality sensor.

2. The system of Claim 1, wherein the air quality sensor comprises a volatile organic compound (VOC) sensor.

3. The system of Claim 1 or Claim 2, wherein the evacuator module further includes a filter arranged in the flow path, and the air quality sensor is positioned upstream of the filter.

4. The system of Claim 3, wherein the evacuator module further includes a flow sensor in communication with the controller and arranged to sense a flow rate of the smoke conveyed to the filter, wherein the parameter comprises an amount of particulate in the smoke, and wherein the controller is further operable to determine a volumetric flow rate of particulate conveyed to the filter based on the amount of particulate in the smoke and the flow rate of smoke conveyed to the filter.

5. The system of Claim 4, wherein the controller is further operable to:
set the motor to a first speed;
compare the volumetric flow rate of particulate conveyed to the filter to a first threshold volumetric flow rate; and
decrease the first speed to a second speed based on the volumetric flow rate of particulate conveyed to the filter being less than the first threshold volumetric flow rate.

6. The system of Claim 5, wherein the controller is further operable to:
compare the volumetric flow rate of particulate conveyed to the filter to a second threshold volumetric flow rate based on the volumetric flow rate of particulate conveyed to the filter being greater than the first threshold volumetric flow rate; and
set the motor to a third speed greater than the first speed based on the volumetric flow rate of particulate conveyed to the filter being greater than the second threshold volumetric flow rate;
optionally wherein the controller is further operable to maintain the first speed based on the volumetric flow rate of particulate conveyed to the filter being less than the second threshold volumetric flow rate.

7. The system of Claim 6, wherein the second threshold volumetric flow rate is greater than the first threshold volumetric flow rate.

8. The system of Claim 1, wherein the evacuator module further includes a filter arranged in the flow path, and the air quality sensor is positioned downstream of the filter.

9. The system of Claim 8, wherein the evacuator module further includes a flow sensor in communication with the controller and arranged to sense a flow rate of filtered air discharged from the filter, wherein the parameter comprises an amount of particulate in the filtered air, and wherein the controller is further operable to determine a volumetric flow rate of particulate in the filtered air based on the amount of particulate in the filtered air and the flow rate of the filtered air discharged from the filter;
optionally wherein the controller is further operable to:
compare the volumetric flow rate of particulate in the filtered air to a threshold volumetric flow rate; and
prevent the motor from driving the pump based on the volumetric flow rate exceeding the threshold volumetric flow rate;
optionally wherein the controller is further operable to determine an effectiveness of the filter.

10. A system, comprising:
an evacuator module that provides:
a housing;
a first inlet port defined in the housing and facilitating fluid communication with a surgical site;
a second inlet port defined in the housing and facilitating fluid communication with an ambient environment;
a three-way valve arranged within the housing and including:
a first inlet in fluid communication with the first inlet port via a first flow path;
a second inlet in fluid communication with the second inlet port via a second flow path; and
an outlet; and
a pump drivable by a motor and in fluid communication with the outlet via a third flow path.

11. The system of Claim 10, wherein the three-way valve is transitionable between:
a first state, in which the surgical site is fluidically coupled to the pump via the first and third flow paths, and the ambient environment is fluidically decoupled from the pump; and
a second state, in which the ambient environment is fluidically coupled to the pump via the second and third flow paths, and the surgical site is fluidically decoupled from the pump.

12. The system of Claim 11, further comprising a controller operable to transition the three-way valve between the first and second states.

13. The system of Claim 12, wherein the controller is in operable communication with the motor and further operable to:
transition the three-way valve to the first state to allow the pump to draw smoke generated by a surgical instrument from the surgical site;
transition the three-way valve to the second state to allow the pump to draw ambient air from the ambient environment based on the surgical instrument ceasing to generate smoke.

14. The system of Claim 13, wherein the evacuator module further includes a filter arranged in the third flow path, and the controller is further operable to determine a status of the filter based on the pump drawing ambient air from the ambient environment.

15. The system of Claim 14, wherein the evacuator module further includes:
a first pressure sensor arranged upstream of the filter to measure a first pressure; and
a second pressure sensor arranged downstream of the filter to measure a second pressure,
wherein the controller is configured to determine a pressure differential across the filter based on the first and second pressures, and
wherein the status of the filter is determined by comparing the pressure differential to a threshold pressure differential;
optionally wherein the controller is configured to prevent the motor from driving the pump based on the pressure differential exceeding the threshold pressure differential.
